# EUROPEAN PATENT SPECIFICATION

(11) **EP 0 503 035 B1**
(45) Date of publication and mention of the grant of the patent: **12.12.2001**
(21) Application number: 91917288.2
(22) Date of filing: 20.09.1991
(51) Int. Cl.: C07C 401/00, A61K 31/59

(54) **NOVEL 1ALPHA-HYDROXY VITAMIN D4 AND NOVEL INTERMEDIATES AND ANALOGUES**
NEUES 1ALPHA-HYDROXY VITAMIN-D4 UND NEUE ZWISCHENPRODUKTE UND ANALOGE
NOUVELLE 1ALPHA-HYDROXY VITAMINE D 4 ET NOUVEAUX INTERMEDIAIRES ET ANALOGUES

(30) Priority: 21.09.1990 US 586854
(43) Date of publication of application: 16.09.1992
(60) Divisional application: 99104552.7
(73) Proprietor: BONE CARE INTERNATIONAL, INC., Madison, WI 53711 (US)
(72) Inventor: KNUTSON, Joyce, C., Madison, WI 53705 (US); BISHOP, Charles, W., Verona, WI 53573 (US); MORIARTY, Robert, M., Oak Park, IL 80302 (US)
(74) Representative: Ford, Michael Frederick
(86) International application number: PCT/US91/06865
(87) International publication number: WO 92/05130

(56) References cited:
- EP-A- 0 390 097
- WO-A-87/00834
- WO-A-90/10620
- BE-A- 877 356
- US-A- 4 202 859
- BULLETIN OF THE CHEMICAL SOCIETY OF JAPAN, vol.63, no.8, August 1990, TOKYO, JP pages 2233 - 2238 M. TSUJI ET AL 'Syntheses of 22,23-dihydroxyvitamin D2 and its 24R-epimer, new vitamin D2 derivatives'
- CHEMICAL ABSTRACTS, vol. 113, no. 1, 2 July 1990, Columbus, Ohio, US; abstract no. 6683y, Y. TACHIBANA 'Preparation of 1beta-hydroxyvitamin D2 and D3.' page 6688 ;column 2 ; & JP-A-02 011 563 (NISSHIN FLOUR MILLING CO.)
- CHEMISTRY LETTERS, no.8, August 1985, TOKYO, JP pages 1131 - 1132 H. NEMOTO ET AL 'A stereoselective synthesis of 1 alpha-hydroxy-vitamin D3'
- DELUCA et al. Arch. Biochem. and Biophys. 124, 122-128 (1968) Synthesis, Biological Activity and Metabolism of 22,23 3H Vitamin D4.
- WINDAVS et al. 2. Physiol. Chem. 247, 1937, pp. 185 to 188. Ueber das Krystallisierte Vitamin D4.

## Description

This invention relates to biologically active vitamin D₄ compounds. More specifically, this invention relates to 1α-hydroxy vitamin D₄ and intermediates used in its synthesis, and 1,24 dihydroxy vitamin D₄.

This invention also relates to a pharmaceutical composition which includes a pharmaceutically effective amount of a 1α-hydroxy vitamin D₄ compound, and to a method of controlling abnormal calcium metabolism by administering a pharmaceutically effective amount of such a compound.

Vitamin D is known to be important in the regulation of calcium metabolism in animals and man. See, Harrison's Principals of Internal Medicine: Part Eleven, "Disorders of Bone and Mineral Metabolism, Chapter 335," E. Braunwald, et al., (eds.), McGraw-Hill, New York, 1987, pp. 1860-1865. The two most commonly known, useful forms of vitamin D are vitamin D₃ and vitamin D₂. Vitamin D₃ is synthesized endogenously in the skin of animals and man, whereas vitamin D₂ is the form of vitamin D supplied by plants. Vitamin D₂ differs from vitamin D₃ in that it contains a double bond between C22 and C23 and further contains a C24-methyl group. In man and rats, vitamin D₃ and vitamin D₂ have equivalent biopotency.

Vitamin D₄, also known as irradiated 22,23-dihydro ergosterol or 22,23-dihydro vitamin D₂ or 22,23-dihydroergocalciferol, differs from vitamin D₃ in that it contains a C24 methyl group. Vitamin D₄ was-first described in 1936. See, Grab, W., Z.Physiol. Chem., 243:63 (1936); McDonald, F.G., J. Biol. Chem., 114:IVX (1936). See also, Windaus, A. and Trautmann, G., Z. Physiol. Chem., 247:185-188 (1937). These references report some disagreement as to the level of biological activity of the vitamin suggesting that in the rat, vitamin D₄ is one-third.or three-fourths as active as vitamin D₃ and in the chick, either one-tenth or one-fifth as active as vitamin D₃.

A more definitive study of the biological activity of vitamin D₄ was made by DeLuca, et al., in 1968. DeLuca, et al., Arch. Biochem. Biophys., 124:122-128 (1968). There, the authors confirmed that vitamin D₄ was less active than vitamin D₃. DeLuca, et al., report that, in their hands, vitamin D₄ is two-thirds as active as vitamin D₃ or vitamin D₂ in the rat, and one-fifth as active as vitamin D₃ in the chick.

DeLuca, et al., make reference to the fact that "[t]he synthesis of vitamin D₄ has apparently been little used since it was first described by Windhaus and Trautmann," and comment, "[t]his is perhaps due to the fact that vitamin D₄ is only of academic interest."

To applicants' knowledge, vitamin D₄ has remained "only of academic interest" as applicants are unaware of any further study of vitamin D₄ since that reported by DeLuca, et al. In fact, The Merck Index states with respect to vitamin D₄, "Its biological activity seems doubtful." Merck Index, S. Budavari (ed.), 11th ed., Merck & Co., Rahway, N.J., (1989) pp. 1579, #9930.

Bull. Chem. Soc. Jpn., 63, 2233-2238 (1990) reports a synthetic route to 1α,25-dihydroxy vitamin D₄. This compound was synthesized with a view to investigating the influence of the C-22 double bond on the physiological activity of vitamin D₂.

Since DeLuca, et al., discovered the active form of vitamin D₃, 1,25-dihydroxy vitamin D₃, (U.S. Patent No. 3,697,559) and its synthetic precursor, 1α-hydroxy vitamin D₃, (U.S. Patent 3,741,996), most interest has centered on developing therapeutic uses of these active vitamin D₃ metabolites. Unfortunately, while the vitamin D₃ metabolites held great promise as therapeutic agents, this promise has never been fully realized because of the extreme toxicity of these agents. For example, toxicity limits the efficacy of vitamin D₃, its active forms and analogs, to prevent bone loss or restore lost bone. Many studies indicate that at dosages required for these agents to be effective in bone loss prevention or restoration, hypercalcemia and hypercalciuria are problems. It has been reported that 1α-hydroxy vitamin D₃ at a daily dose of 2 µg/day (which has been shown in some studies to be effective in preventing loss of bone) causes toxicity in approximately 67% of patients. What is needed is a biopotent vitamin D metabolite of low toxicity, such that the drug is practical as a

The novel compounds of the invention, 1α-hydroxy vitamin D₄, and 1,24-dihydroxy vitamin D₄, are bioactive forms of vitamin D₄. The present inventors have. discovered that these active forms of vitamin D₄ and also 1,25-dihydroxy vitamin D₄ display much greater biopotency than would be predicted on the basis of the previously reported bioassays of vitamin D₄. The present inventors have also discovered, that the bioactive compounds are less toxic than would be predicted on the basis of their biopotency. This combination of high activity with low toxicity makes the compounds of the invention useful as therapeutic agents in the treatment of disorders of calcium metabolism. The compounds are advantageously used as the active compounds of pharmaceutical compositions for diseases induced by abnormal metabolism of calcium.

In order to study the novel compounds of the invention, it was necessary to develop processes for their production. 1-alpha-hydroxy vitamin D₄ was made synthetically and in the course of that synthesis, novel intermediates were also produced. 1,25-dihydroxy vitamin D₄ and 1,24-dihydroxy vitamin D₄ are isolated as biological products of the metabolism of 1α-hydroxy vitamin D₄.

Other advantages and a fuller appreciation of the specific adaptations, compositional variations, and physical and chemical attributes of the present invention will be gained upon an examination of the following detailed description of the invention, taken in conjunction with the accompanying drawings.

The present invention will hereinafter be described in conjunction with the appended drawings, wherein like designations refer to like elements throughout and in which:
Figure 1 illustrates preparative steps for the synthesis of vitamin D₄; and
Figure 2 illustrates preparative steps for the synthesis of 1α-hydroxy vitamin D₄ starting with vitamin D₄.

The present invention provides synthetic 1α-hydroxy vitamin D₄ (1α-OH-D₄) compounds as well as tosylated and cyclic derivatives of vitamin D₄.

As used herein, the terms "biological activity" or "biologically active" are meant to refer to biochemical properties of compounds such as affecting metabolism, e.g., affecting serum calcium concentration, or binding to an appropriate receptor protein, e.g., binding to vitamin D recepter protein.

In one of its aspects, the invention encompasses biologically active compounds of the general formula (I): 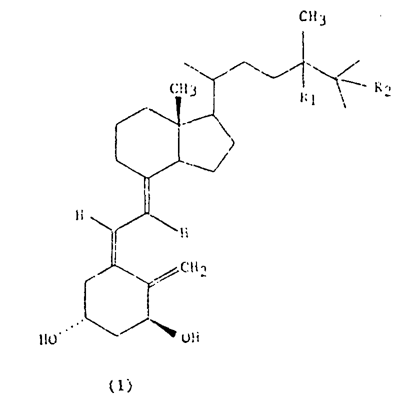 wherein R₁ is either H or OH, and R₂ is either H or OH, and salts, hydrates and solvates thereof and wherein the compound is not 1,25 dihydroxy vitamin D₄. Preferred compounds among those of formula (I) are those in which R₁ and R₂ are both H; and R₁ = OH and R₂ = H.

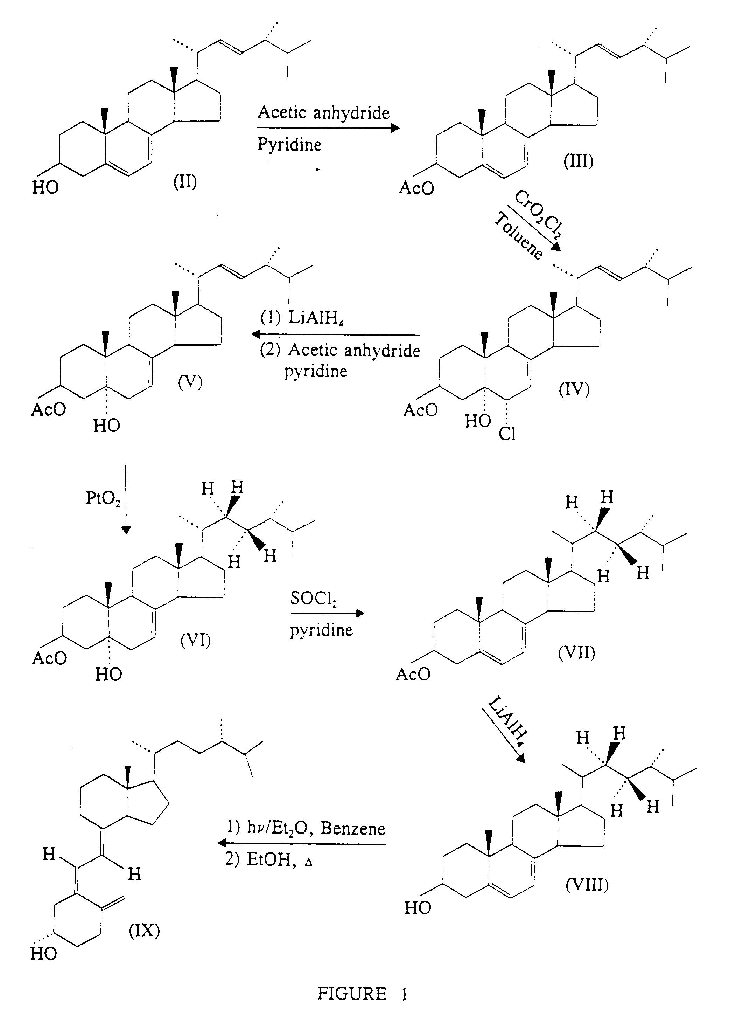

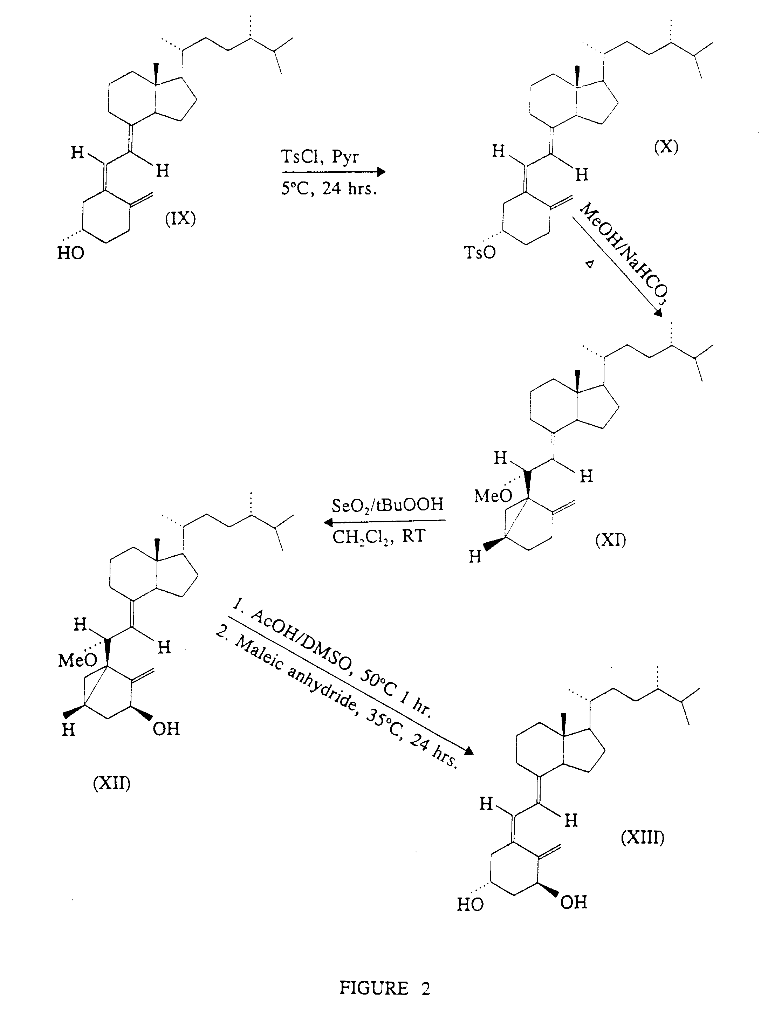

In another aspect, the invention involves the preparation of compounds of formula (I). Synthesis of 1α-hydroxy vitamin D₄, i.e., compounds of formula (I) wherein R₁ and R₂ are H, is accomplished according to the schema presented in Figures 1 and 2. As seen in Figure 1, the synthesis uses ergosterol as the starting material. Ergosterol undergoes side chain saturation in a six-step process to yield 22,23-dihydroergosterol (VIII) using a procedure similar to that of Barton, et al., JCS Perkin I, 1976, 821-826. The 22,23-dihydroergosterol is then irradiated as described in Windaus, et al., Z. Physiol. Chem., 1937, 147:185, to yield vitamin D₄ [22,23-dihydroergocalciferol] (IX). As seen in Figure 2, vitamin D₄ is then hydroxylated in a four-step process to yield 1α-hydroxy vitamin D₄ using a procedure similar to that described by Paaren, et al., J. Org. Chem., 1980, 45:3253.

Specifically, ergosterol is acetylated to form the 3β-acetate. This ergosterol acetate is subjected to hydroxyhalogenation at the 5,6 double bond to form the 6α-chloro-5α-hydroxy derivative. This chlorohydrin is reduced and reacetylated to the 5α-hydroxy (i.e., 5α-ol) derivative. The 5α-ol is subjected to hydrogenation to saturate the side chain. The resulting 3β-acetoxyergost-7en-5α-ol is reduced to 22,23 dihydroergosterol acetate which is in turn reduced to yield 22,23 dihydroergosterol. The 22,23 dihydroergosterol is then irradiated to form vitamin D₄. Vitamin D₄ is then tosylated to yield 3β-tosyl vitamin D₄. The tosylate is displaced by solvolysis to yield the 6-methoxy-3, 5-cyclovitamin D₄. The cyclovitamin D₄ is subjected to allyllic oxidation to form the 1α-hydroxy cyclovitamin derivative. The 1α-hydroxy cyclovitamin derivative is sequentially solvolyzed and subjected to a Diels-Alder-type reaction which removes the 5-methoxy group and separates the 1α-hydroxy vitamin D₄ (5,6-cis) from the 5,6 trans-1α-hydroxy vitamin D₄.

The 1,24 dihydroxy vitamin D₄ and 1,25 dihydroxy vitamin D₄ metabolites of 1α-hydroxy vitamin D₄, are synthesized by incubating the 1α-hydroxy derivatives with human liver cells, culturing the cells, and recovering the 1,24 dihydroxy or 1,25 dihydroxy vitamin D₄. Using vitamin D receptor' protein binding tests, these metabolites are determined to be biologically active.

According to a further aspect of-the invention there is provided a prophylactic or therapeutic pharmaceutical composition, comprising an amount of a compound of the formula (I'): 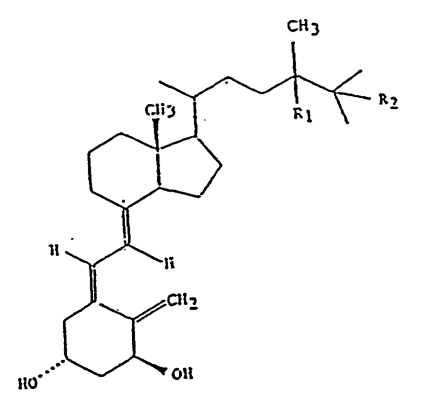 wherein R₁ is either H or OH and R₂ is either H or OH in combination with a pharmaceutically acceptable vehicle.

The compounds of formula (I') have been found to possess valuable pharmacological activity, namely, as controlling agents for calcium metabolism, especially serum calcium concentrations. Specifically, the compounds of formula (I') increase serum calcium concentrations in rats with vitamin D deficiency. It has also been found that the compounds of formula (I') have low toxicity, which enhances their pharmaceutical properties. Compounds of formula (I') have a toxicity, as measured by the LD₅₀ test, which is similar to that of corresponding vitamin D₂ compounds and lower than that of corresponding vitamin D₃ compounds. Thus, the compounds of the invention are applicable to various clinical and veterinary fields, and are particularly useful for the treatment of abnormal metabolism of calcium and phosphorus.

Compounds of formula (I') can be used in methods of controlling calcium metabolism, such as for treating abnormal calcium metabolism caused, e.g., by liver failure, renal failure, gastrointestinal failure, etc. The compounds of formula (I') can be used to treat prophylactically or therapeutically vitamin D deficiency diseases and related diseases, for example, renal osteodystrophy, steatorrhea, anticonvulsant osteomalacia, hypophosphatemic vitamin D-resistant rickets, osteoporosis, including postmenopausal osteoporosis, senile osteoporosis, steriod-induced osteoporosis, and other disease states characteristic of loss of bone mass, pseudodeficiency (vitamin D-dependent) rickets, nutritional and malabsorptive rickets, osteomalacia and osteopenias secondary to hypoparathyroidism, post-surgical hypoparathyroidism, idiopathic hypoparathyroidism, pseudohypoparathyroidism, and alcoholism. The compounds of formula (I), preferably those wherein R1 or R2 is OH, such as 1α,24 dihydroxy vitamin D₄, are of value for the treatment of hyperproliferative skin disorders such as psoriasis.

The compounds of formula (I') are useful as active compounds in pharmaceutical compositions having reduced side effects and low toxicity as compared with the known analogs of active forms of vitamin D₃, when applied, for example, to diseases induced by abnormal metabolism of calcium.

The pharmacologically active compounds of this invention can be processed in accordance with conventional methods of pharmacy to produce medicinal agents for administration to patients, e.g., mammals including humans. For example, the compounds of formula (I') can be employed in admixtures with conventional excipients, e.g., pharmaceutically acceptable carrier substances suitable for enteral (e.g., oral), parenteral, or topical application which do not deleteriously react with the active compounds.

Suitable pharmaceutically acceptable carriers include but are not limited to water, salt solutions, alcohols, gum arabic, vegetable oils (e.g., corn oil, cottonseed oil, peanut oil, olive oil, coconut oil), fish liver oils, oily esters such as Polysorbate 80, polyethylene glycols, gelatine, carbohydrates (e.g., lactose, amylose or starch), magnesium stearate, talc, silicic acid, viscous paraffin, fatty acid monoglycerides and diglycerides, pentaerythritol fatty acid esters, hydroxy methylcellulose, polyvinyl pyrrolidone, etc.

The pharmaceutical preparations can be sterilized and, if desired, be mixed with auxiliary agents, e.g., lubricants, preservatives, stabilizers, wetting agents, emulsifiers, salts for influencing osmotic pressure, buffers, coloring, flavoring and/or one or more other active compounds, for example, vitamin D₃ or D2 and their 1α-hydroxylated metabolites, conjugated estrogens or their equivalents, anti-estrogens, calcitonin, biphosphonates, calcium supplements, cobalomin, pertussis toxin and boron.

For parenteral application, particularly suitable are injectable, sterile solutions, preferably oily or aqueous solution, as well as suspensions, emulsions, or implants, including suppositories. Ampoules are convenient unit dosages.

For enteral application, particularly suitable are tablets, dragees, liquids, drops, suppositories, lozenges, powders, or capsules. A syrup, elixir, or the like can be used if a sweetened vehicle is desired.

Sustained or directed release compositions can also be formulated, e.g., liposomes or those in which the active compound is protected with differentially degradable coatings, e.g., by microencapsulation, multiple coatings, etc.

For topical application, suitable nonsprayable viscous, semi-solid or solid forms can be employed which include a carrier compatible with topical application and having a dynamic viscosity preferably greater than water. Suitable formulations include, but are not limited to, solutions, suspensions, emulsions, creams, ointments, powders, liniments, salves, aerosols, transdermal patches, etc., which are, if desired, sterilized or mixed with auxiliary agents, e.g., preservatives, stabilizers, demulsifiers, wetting agents, etc.

For rectal administration, compounds are formed into a pharmaceutical composition containing a suppository base such as cacao oil or other triglycerides. To prolong storage life, the composition advantageously includes an antioxidant such ascorbic acid, butylated hydroxyanisole or hydroquinone.

Oral administration of the pharmaceutical compositions of the present invention is preferred. Generally, the compounds of this invention are dispensed by unit dosage form comprising about 0.5 µg to about 25 µg in a pharmaceutically acceptable carrier per unit dosage. The dosage of the compounds according to this invention generally is about 0.01 to about 0.5 µg/kg/day, preferably about 0.04 to about 0.3 µg/kg/day.

It will be appreciated that the actual preferred amounts of active compound in a specific case will vary according to the efficacy of the specific compound employed, the particular compositions formulated, the mode of application, and the particular situs and organism being treated. For example, the specific dose for a particular patient depends on the age, body weight, general state of health, sex, on the diet, on the timing and mode of administration, on the rate of excretion, and on medicaments used in combination and the severity of the particular disorder to which the therapy is applied. Dosages for a given host can be determined using conventional considerations, e.g., by customary comparison of the differential activities of the subject compounds and of a known agent, such as by means of an appropriate conventional pharmacological protocol.

In a still further aspect, the compounds of formula (I') can also be advantageously used in veterinary compositions, for example, feed compositions for domestic animals to treat or prevent hypocalcemia. Generally, the compounds are dispensed in animal feed such that normal consumption of such feed provides the animal about 0.01 to about 0.5 µg/kg/day.

The following examples are to be construed as merely illustrative, and not limitative of the remainder of the disclosure in any way whatsoever. In the following examples, all temperatures are set forth in degrees Celsius; unless otherwise indicated, all parts and percentages are by weight. Proton nuclear magnetic (¹H NMR) spectra were recorded with an IBM Sy-200(200 mHz) and a Bruker Am--400(400 mHz) with aspect 3000 Computer in CDCl₃ solutions with CHCl₃ as an internal standard. Infrared spectra were recorded with a Fourier transform (FTIR) using samples as potassium bromide (KBr) pellets or as liquids. Mass spectra were recorded with a Finnigan MAT-90 mass spectrometer at 20 eV/CI. Melting points are determined on a Hoover-Thomas (capillary) Uni-Melt and a Fisher-Johns melting point apparatus (cover-slip type).

### Example 1: Synthesis of 1α-hydroxy vitamin D₄.

Ergosterol (II) was converted to ergosterol acetate (III) by dissolving 100 g (0.25 mol) ergosterol in 600 ml of anhydrous pyridine and 68 ml (0.7 mol) acetic anhydride. The solution was stirred overnight at room temperature after which time the solution was cooled by adding 1.2 L ice, causing a precipitate to form. The precipitate was washed five times with 400 ml portions of water, then once with 400 ml of CH₃CN. The resulting product was air dried to yield 79 g (71%) of ergosterol acetate , as a white crystalline solid and had the following characteristics: melting point (m.p.): 16.9-171°C; ¹H NMR: (400 MHz, CDCl₃), δppm 2.05 (3H, s, 3β-CH₃CO), 4.65-4.75 (1H, m, 3α-H) 5.15-5.25 (2H, m, 22-H and 23-H); 5.4 (1H, d, 6-H), 5.6 (1H, d, 7-H); FTIR [KBr]: 1734 cm⁻¹ (C=O stretching) 968 cm⁻¹ (C-H bending).

Ergosterol acetate (III) (26 gm, 0.062 M) was dissolved in 2.5 L of freshly distilled deoxygenated toluene. To this solution 9 ml (0.111 mol) chromyl chloride dissolved in 240 ml dry CH₂Cl₂ was added under nitrogen at -78°C over a thirty minute period. The reaction system was stirred at -78°C for an additional fifteen minutes, and then 62 ml of a saturated solution of sodium borohydride in ethanol was added in one portion. After stirring at -78°C for an additional fifteen minutes, the reaction solution was poured into a two phase system of 3N hydrochloric acid (3L) and benzene (3L). The organic layer was separated, then washed with water (2L), twice with a brine solution (2 x 1L) and then dried with anhydrous MgSO₄. The dried solution was filtered and concentrated in vacuo. The crude crystalline product was then treated with CH₃CN (280ml) and filtration of the thus formed slurry yielded 12.5 g (41%) of white crystalline 3β-acetoxy-6α-chloroergosta-7,22-dien-5α-ol (IV) and had the following characteristics: m.p.: 190-192°C; ¹H NMR: (400 MHz, CDCl₃), δppm 2.05 (3H, s, 3β-OAc), 4.65 (1H, d, 6β-H), 5.1 (1H, s, 7-H), 5.1-5.3 (2H, m, 22-H and 23-H); FTIR [KBr]: 1732 cm⁻¹ (C=O stretching), 968 cm⁻¹ (C-H bending), 3437 cm⁻¹ (O-H stretching).

The 3β-acetoxy 6α-chloroergosta-7,22-dien-5a-ol (IV) (21.4 g, 0.044 mol) in dry THF (900 ml) was added slowly to a stirred suspension of lithium aluminium hydride (2.66 g, 0.07 mol) in dry THF (750 ml) at room temperature under nitrogen. The mixture was refluxed for three hours and cooled to O°C. Excess hydride was decomposed with saturated Na₂SO₄ solution. Filtration through anhydrous Na₂SO₄ and evaporation of the filtrate gave a solid, which was treated directly with acetic, anhydride (110 ml) and dry pyridine (220 ml) at O°C. Removal of solvent under reduced pressure yielded the acetate (12.75 g, 61%), 3β-acetoxyergosta-7,22-dien-5α-ol (V) and had the following characteristics: m.p.: 229-232°C; FTIR [KBr] 1736 cm⁻¹ (C=O stretching), 3460 cm⁻¹ (O-H stretching), 972 cm⁻¹ (C-H bending).

3β-Acetoxyergosta-7,22-dien-5α-ol (V) (2.5 g, 0.0055 mol) was shaken for sixteen hours with freshly prepared PtO₂ (0.5 g) in ethyl acetate (820 ml) under H₂ gas (15 psi). The catalyst was removed by filtration and evaporation of the filtrate gave the crude acetate which was dissolved in CH₂Cl₂ and chromatographed on silica gel. Elution with CH₂Cl₂ gave substantially pure 3β-acetoxyergost-7-en-5a-ol (VI) (2.15 g, 85%) as a white crystalline material and had the following characteristics: m.p.: 228-232°C; ¹H NMR: (400 MHz, CDCl₃), δppm 2.05 (3H, s, 3β-OAc), 5.05-5.20 (2H, m, 3α-H and 7-H); FTIR [KBr]: 1736 cm⁻¹ (C=O stretching), 3462 cm⁻¹ (O-H stretching).

Redistilled thionyl chloride (9.7 ml) in dry pyridine (170 ml) was added to compound 3β-acetoxyergost-7-en-5α-ol (VI) (12.0 g, 0.0262 mol) in dry pyridine (800 ml) at 0°C under nitrogen. After 2.5 hours, the solution was diluted with ice cold H₂O (1.5 L) and extracted with two portions of ether (2.5 L + 1.5L). The combined ether extracts were washed with a NaHCO₃ solution (1.0 L x 2), then 1N HCl (1.5 L x 2) and then water (1 L). The ether solution was dried with MgSO₄, and after filtration, evaporated under reduced pressure to yield a crude product which was converted to a slurry with CH₃CN (100 ml). The product was collected by filtration and recrystallized from CH₃CN to yield 4.5 g. (39%) of a white crystalline 22,23-dihydroergosteryl acetate (VII) and had the following characteristics: m.p.: 144-147°C; ¹H NMR: (400 MHz, CDCl₃), δppm 2.05 (3H, s, 3β-OAc), 4.65-4.75 (1H, m, 3α-H), 5.4 (1H, d, 6-H), 5.6 (1H, d, 7-H); FTIR [KBr]: 1734 cm⁻¹ (C=O stretching).

22,23-Dihydroergosteryl acetate (VII) (4.8 g, 0.011 mol) was added at once to a stirred suspension of lithium aluminium hydride (2.5 g, 0.066 mol) in dry ether (1.1 L) at room temperature. The mixture was stirred for two hours at room temperature. 5N NaOH was added to destroy excess lithium aluminium hydride and H₂O (500 ml) was then added. The aqueous solution was then extracted with four 250 ml portions of ether. The combined ether extracts and combined organic layer were washed with brine solution (1 L), then dried with Na₂SO₄. Evaporation of ether under reduced pressure gave the compound, 22,23-dihydroergosterol, (VIII) (4.1 g, 94%) as a white crystalline material and had the following characteristics: m.p.: 147-150°C; ¹H NMR: (400 MHz, CDCl₃), δppm 3.6-3.7 (1H, m, 3α-H), 5.4 (1H, d, 6H), 5.6 (1H, d, 7-H); FTIR [KBr]: 3400 cm⁻¹ (O-H stretching).

22,23-Dihydroergosterol (VIII) (2.0 g, 5.0 mmol) was dissolved in a solution of diethyl ether and benzene (4:1, 600 ml) and irradiated (Hannovia immersion lamp, 450 watts) with stirring under argon in a water-cooled quartz vessel for three hours. The solution was concentrated in vacuo to yield a gummy solid, which was redissolved in 100 ml. of ethanol and heated at reflux under argon for eight hours. Then, the solution was concentrated in vacuo and the residue was adsorbed on a silica gel column and eluted with 30% ethyl acetate in hexane to afford vitamin D₄ (22,23-dihydroergocalciferol) (IX) with a yield of 1.2 g. (60%) and with the following characteristics: ¹H NMR: (400 MHz, CDCl₃), δppm 0.55 (3H, s, 18-H₃) 0.78 (6H, dd, 26-H₃ and 27-H₃) 0.87 (3H, d, 21-H₃) 0.93 (3H, d, 28-H₃) 3.94 (1H, m, 3-H) 4.82 (1H, m (sharp), 19-H), 5.04 (1H, m (sharp), 19-H), 6.04 (1H, d, 7-H) 6.24 (1H, d, 6-H).

To a stirred solution of vitamin D₄ (IX) (3.0 g, 7.5 mmol) in 10 ml of dry pyridine was added freshly recrystallized p-toluenesulfonyl chloride (3.6 g, 19 mmol) at 0°C. The reaction mixture was stirred at 5°C for 24 hours, and was then quenched by pouring the mixture over ice and saturated NaHCO₃ (100 ml) with stirring. The aqueous suspension was extracted with CH₂Cl₂ (3 x 300 ml). The combined organic extracts were washed with 10% HCl (3 x 200 ml), saturated NaHCO₃ (3 x 200 ml) and saturated NaCl (2 x 200 ml), dried over MgSO₄ and concentrated in vacuo to yield 3.5 g. (84%) of the novel intermediate compound vitamin D₄ tosylate (X) and had the following characteristics: ¹H NMR (400 MHz, CDCl₃), δppm 0.54 (3H, s, 18-H₃) 0.78 (6H, dd, 26-H₃ and 27-H₃) 0.87 (3H, d, 21-H₃), 0.96 (3H, d, 28-H₃) 2.45 (3H, s, CH₃ (tosylate) 4.68 (3H, m, 3-H) 4.82 (1H, m (sharp), 19-H) 5.04 (1H, m (sharp), 19-H), 5.95 (1H, d 7-H), 6.09 (1H, d, 6-H) 7.34 and 7.79 (4H, d, aromatic).

To a stirred suspension of NaHCO₃ (17.0 g, 202 mmol) in methanol (200 ml) a solution of vitamin D₄ tosylate (X) (3.5 g, 6.3 mmol) in dry CH₂Cl₂ (10 ml) was added dropwise. The reaction mixture was refluxed overnight under argon, and then cooled to room temperature and concentrated in vacuo to about 50 ml. The reaction concentrate was diluted with ether (600 ml), washed with water (3 x 300 ml), dried over MgSO₄ and concentrated in vacuo. The residue was passed through a silica gel column and eluted with 10% ethyl acetate in hexane to afford the novel intermediate compound 3,5 cyclovitamin D₄ (XI) (heavy oil) with a yield of 1.5 g. (58%) and had the following characteristics: ¹H NMR (400 MHz, CDCl₃), δppm 0.56 (3H, s, 18-H₃) 0.78 (6H, dd, 26-H₃ and 27-H₃), 0.87 (3H, d, 21-H₃), 0.94 (3H, d, 28-H₃), 3.28 (3H, s, OCH₃) 4.2 (1H, d, 6-H), 4.91 (1H, m (sharp), 19-H), 4.98 (1H, d 7-H), 5.08 (1H, m (sharp), 19-H).

Anhydrous tert-butyl hydroperoxide in toluene (3M) (2.6 ml, 7.8 mmol) was added to a stirred suspension of selenium dioxide (0.22 g, 2 mmol) in dry CH₂Cl₂ (150 ml) in a three necked flask. The mixture was stirred for three hours under argon. Pyridine (0.3 ml, 3.7 mmol) was then added, and cyclovitamin D₄ (XI) (1.5 g, 3.6 mmol) was then introduced as a solution in CH₂Cl₂ (50 ml). After stirring for thirty minutes, 10% aqueous NaOH solution (200 ml) was added. The reaction mixture was then diluted with ether (500 ml) and the phases were separated. The organic phase was washed with 10% NaOH (3 x 200 ml), water (2 x 200 ml) and saturated NaCl solution (2 x 200 ml), dried over MgSO₄ and concentrated in vacuo. The residue was absorbed on a silica gel column and eluted with 30% ethyl acetate in hexane to afford 0.45 g. (29%) of the novel intermediate compound 1α-hydroxy 3,5-cyclovitamin D₄ (XII) (oil) and had the following characteristics: ¹H NMR (400 MHz, CDCl₃), δppm 0.54 (3H, s, 18-H₃) 0.78 (6H, dd, 26-H₃ and 27-H₃) 0.86 (3H, d, 21-H₃) 0.95 (3H, d, 28-H₃) 3.26 (3H, s, OCH₃) 4.2 (1H, d, 6-H), 4.22 (1H, m, 1-H), 4.95 (1H, d 7-H), 5.18 (1H, d, 19-H) 5.25 (1H, d, 19-H).

A solution of 1α-hydroxy 3,5-cyclovitamin D₄ (XII) (0.45 g, 1.05 mmol) in a solution of dimethyl sulfoxide (4.5 ml) and glacial acetic acid (3.6 ml) was heated to 50°C under argon for one hour. The reaction mixture was then poured over ice and saturated NaHCO₃ solution (100 ml), and extracted with ether (3 x 200 ml). The combined ether extracts were washed with saturated NaHCO₃ solution (3 x 200ml), water (3 x 200 ml) and saturated NaCl solution (3 x 200 ml), dried over MgSO₄, concentrated in vacuo to give a mixture containing 5,6-cis and 5,6-trans 1α-hydroxy vitamin D₄ (about 4:1 by ¹H NMR) with a yield of 0.4g, (92%). The mixture of 5,6-cis and 5,6-trans 1α-hydroxy vitamin D₄ (0.4 g, 0.97 mmol) was dissolved in ethyl acetate (25 ml) and treated with freshly recrystallized maleic anhydride (0.08 g, 0.8 mmol). This reaction mixture was heated to 35°C under argon for 24 hours. After evaporation of the solvent in vacuo, the crude mixture was chromatographed over a silica gel column using ethyl acetate and hexane (1:1) as eluent, to afford the novel active form of vitamin D₄, 5,6-cis 1α-hydroxy vitamin D₄ (XIII) with a yield of 90 mg (23%) and had the following characteristics: m.p.: 128-130°C; IR νₘₐₓ (Neat): 3400 cm⁻¹ (OH stretching); ¹H NMR (400 MHz, CDCl₃), δppm 0.55 (3H, s, 18-H) 0.79 (6H, dd, 26-H₃ and 27-H₃) 0.87 (3H, d, 21-H₃) 0.94 (3H, d, 28-H₃), 4.24 (1H, m, 3-H), 4.44 (1H, m, 1-H), 5.02 (1H, m (sharp), 19-H), 5.34 (1H, m (sharp), 19-H), 6.02 (1H, d 7-H), 6.4 (1H, d, 6-H); Mass spectrum [CI] m/e (relative intensity): 415 (M+1, 41%) 397, (M+1-OH 100%), 379 (27%), 135 (22%).

### Example 2: Biological testing of 1α-hydroxy vitamin D₄

Male weanling rats (Holtzman strain, Holtzman Company, Madison, Wisconsin) were fed a vitamin D deficient diet containing adequate calcium (0.47%) and phosphorus (0.3%). Within three to four weeks, this diet induces an extreme vitamin D deficiency characterized by low serum calcium and poor growth. After four weeks on this diet, the rats had serum calcium values less than 7 mg/dl. The rats were then separated into four groups and orally administered either 1α-hydroxy vitamin D₄ in a vehicle such as coconut oil or the vehicle (control) for each of 14 days. Twenty-four hours after the last dose, the rats were killed and the blood calcium measured by a standard laboratory technique. The results of these determinations are shown in Table 1.

**TABLE 1**

| Increase in Serum Calcium Concentration | | | |
|---|---|---|---|
| Compound | Dose (µg/kg/day) | Number of rats | Serum calcium concentration (mg/dl) ± Standard Deviation |
| Control | - | 10 | 6.1±0.48 |
| 1α-OH-D₄ | 0.042 | 8 | 7.1±0.80 |
| 1α-OH-D₄ | 0.250 | 7 | 11.6±0.45 |
| 1α-OH-D₄ | 1.500 | 9 | 12.7±0.37 |

The data of Table 1 indicate that 1α-hydroxy vitamin D₄ is effective at increasing serum calcium in the vitamin D deficient rat and that the response appears to be dose dependent. Surprisingly, the level of the response appears to compare favorably to that reported by Wientroub, et. al., for 1,25 dihydroxy vitamin D₃ administered to vitamin D deficient rats under experimental conditions similar to those described above. See, Wientroub, S., Price, P.A., Reddi, A.H., "The Dichotomy in the Effects of 1,25 dihydroxy vitamin D₃ and 24,25 dihydroxy vitamin D₃ on Bone Gamma-Carboxyglutamic Acid-Containing Protein in Serum and Bone in vitamin D-Deficient Rats," Calcif. Tissue Int. (1987) 40:166-172.

### Example 3: Toxicity tests

The acute oral toxicity of 1α-OH-D₄ in rats was assessed by determining the mean lethal dose (LD₅₀) using a well-known method. Rats were fed a standard laboratory diet for 8-10 weeks. Five animals of each sex were administered one oral dose of 1α-OH-D₄. The animals were observed for 14 days, and the number of deaths noted. The LD₅₀ value was determined to be about 1.0 mg/kg in males and 3.0 mg/kg in females.

For comparison, the LD₅₀ value for 1α-hydroxy vitamin D₂ under the same conditions was found by applicants to be 1.7 and 1.8 mg/kg. in male and female rats, respectively. The toxicity of 1α-hydroxy vitamin D₂ has previously been reported as less than 1α-hydroxy vitamin D₃. Sjoden, G., Smith, C., Lindgren, U., and DeLuca, H.F., Proc. Soc. Experimental Biol. Med., 178:432-436 (1985).

### Example 4: Generation and Isolation of 1,25-dihydroxy vitamin D₄

The 1α-hydroxy vitamin D₄ of the present invention is incubated with cultured human liver cells which metabolize the compound to several products including the metabolite 1,25 dihydroxy vitamin D₄. The 1,25 metabolite is isolated and purified by high pressure liquid chromatography and identified by gas-chromatography-mass spectrometry. Binding studies demonstrate that the 1,25 dihydroxy vitamin D₄ has good binding affinity for the mammalian vitamin D receptor protein indicating it is biologically active. The procedures used are similar to that described by Strugnell, et. al., Biochem. Pharm. Vol. 40:333-341 (1990).

### Example 5: Generation and isolation of 1,24-dihydroxy vitamin D₄

Generation and isolation of 1,24 dihydroxy vitamin D₄ is accomplished as described in Example 4, above. The 1α-hydroxy vitamin D₄ of the present invention is incubated with cultured human liver cells which metabolize the compound to several products including the metabolite 1,24 dihydroxy vitamin D₄. The 1,24 metabolite is isolated and purified using high pressure liquid chromatography and identified by gas-chromatography-mass spectrometry. Binding studies with the new metabolite demonstrate that the metabolite has good binding affinity for the mammalian vitamin D receptor protein which indicates the drug is biologically active.

### Example 6: Hypercalcemia testing

Female rats are fed a commercial diet containing 0.8% calcium (0.8%) and phosphorus (0.6%). The rats are divided into four groups and each group is orally administered daily either 1α-hydroxy vitamin D₄ in a vehicle such as coconut oil or the vehicle (control) alone for 13 weeks. Twenty-four hours after the last dose, the rats are killed and their serum calcium determined by a standard method.

This procedure demonstrates that the serum calcium concentration is unaffected or only slightly elevated at doses of 1α-hydroxy vitamin D₄ up to 2.5 µg/kg/day.

### Example 7: Further biological testing

Male weanling rats are fed a diet deficient in vitamin D and with low calcium (0.02%). After a period of four weeks has elapsed, the rats are divided into four groups and intravenously administered either 1α-hydroxy vitamin D₄ in a vehicle such as ethanol or the vehicle (control) alone. Sixteen hours after administration, the rats are killed and the intestinal calcium transport measured by using everted duodenal sacs, following the method of Martin and DeLuca, Am. J. Physiol. 216:1352-1359.

Following this procedure demonstrates stimulation of intestinal calcium transport in a dose dependent manner.

### Example 8:

A clinical study is conducted with postmenopausal osteoporotic outpatients having ages between 55 and 75 years. The study involves up to 120 patients randomly divided into three treatment groups, and continues for 12 to 24 months. Two of the treatment groups receive constant dosages of 1α-hydroxy vitamin D₄ (u.i.d.; two different dose levels above 3.0 µg/day) and the other group receives a matching placebo. All patients maintain a normal intake of dietary calcium (500 to 800 mg/day) and refrain from using calcium supplements. Efficacy is evaluated by pre- and post-treatment comparisons of the patient groups with regard to (a) total body, radial, femoral and/or spinal bone mineral density as determined by x-ray absorptiometry (DEXA), (b) bone biopsies of the iliac crest, and (c) determinations of serum osteocalcin. Safety is evaluated by comparisons of urinary hydroxyproline excretion, serum and urine calcium levels, creatinine clearance, blood urea nitrogen, and other routine determinations.

This study demonstrates that patients treated with 1α-hydroxy vitamin D₄ exhibit significantly higher total body, radial, femoral and/or spinal bone densities relative to patients treated with placebo. The treated patients also exhibit significant elevations in serum osteocalcin. Bone biopsies from the treated patients show that 1α-hydroxy vitamin D₄ stimulates normal bone formation. The monitored safety parameters confirm an insignificant incidence of hypercalcemia or hypercalciuria, or any other metabolic disturbance with 1α-hydroxy vitamin D₄ therapy.

### Example 9:

A clinical study is conducted with healthy postmenopausal women having ages between 55 and 60 years. The study involves up to 80 patients randomly divided into two treatment groups, and continues for 12 to 24 months. One treatment group receives a constant dosage of 1α-hydroxy vitamin D₄ (u.i.d.; a dose level above 3.0 µg/day) and the other receives a matching placebo. The study is conducted as indicated in Example 2 above.

This study demonstrates that patients treated with 1α-hydroxy vitamin D₄ exhibit reduced losses in total body, radial, femoral and/or spinal bone densities relative to baseline. values. In contrast, patients treated with placebo show significant losses in these parameters relative to baseline values. The monitored safety parameters confirm the safety of long-term 1α-hydroxy vitamin D₄ administration at this dose level.

### Example 10:

A twelve-month double-blind placebo-controlled clinical trial is conducted with thirty men and/or women with renal disease who are undergoing chronic hemodialysis. All patients enter an eight-week control period during which time they receive a maintenance dose of vitamin D₃ (400 IU/day). After this control period, the patients are randomized into two treatment groups: one group receives a constant dosage of 1α-hydroxy vitamin D₄ (u.i.d.; a dosage greater than 3.0 µg/day) and the other group receives a matching placebo. Both treatment groups receive a maintenance dosage of vitamin D₃, maintain a normal intake of dietary calcium, and refrain from using calcium supplements. Efficacy is evaluated by pre- and post-treatment comparisons of the two patient groups with regard to (a) direct measurements of intestinal calcium absorption, (b) total body radial, femoral and/or spinal bone mineral density, and (c) determinations of serum calcium and osteocalcin. Safety is evaluated by regular monitoring of serum calcium.

Analysis of the clinical data shows that 1α-hydroxy vitamin D₄ significantly increases serum osteocalcin levels and intestinal calcium absorption, as determined by measurements using a singie or double-isotope technique. Patients treated with this compound show normalized serum calcium levels, stable values for total body, radial, femoral and/or spinal bone densities relative to baseline values. In contrast, patients treated with placebo show frequent hypocalcemia, significant reductions in total body, radial, femoral and/or spinal bone density. An insignificant incidence of hypercalcemia is observed in the treated group.

While the present invention has now been described and exemplified with some specificity, those skilled in the art will appreciate the various modifications, including variations, additions, and omissions, that may be made in what has been described. Accordingly, it is intended that these modifications also be encompassed by the present invention and that the scope of the present invention be limited solely by the broadest interpretation that lawfully can be accorded the appended claims.

## Claims (Claims for the following Contracting State(s): DE, DK, FR, GB, IT, NL)

1. The compound of the formula (I): 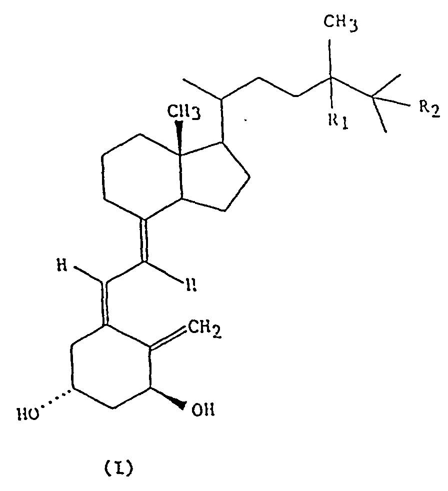 wherein R₁ is either H or OH and R₂ is either H or OH and salts, hydrates and solvates thereof and wherein the compound is not 1,25 dihydroxy vitamin D₄.

2. The compound of claim 1, wherein said compound is 1α-hydroxy vitamin D₄.

3. The compound of claim 1, wherein said compound is 1,24 dihydroxy vitamin D₄.

4. The compound of claim 1, wherein said compound is biologically active.

5. The compound of any one of the preceding claims wherein said compound exhibits biological activity approaching that of 1,25 vitamin D₃ and wherein said compound is less toxic than 1α-hydroxy vitamin D₃ as determined by comparative LD₅₀ values in rats.

6. The vitamin D₄ tosylate compound of the formula (X) :

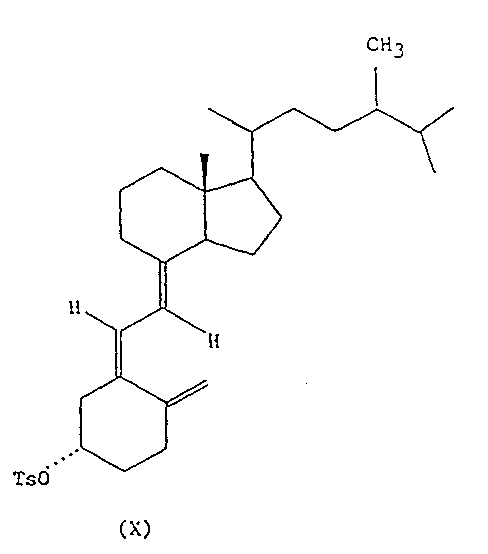

7. The 3,5 cyclovitamin D₄ compound of the formula (XI) :

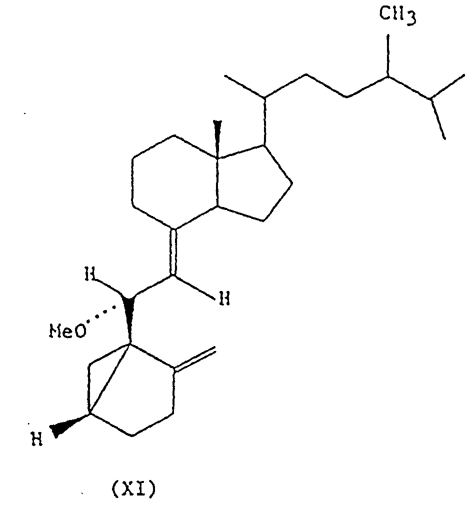

8. The 1α-hydroxy 3,5 cyclovitamin D₄ of the formula (XII):

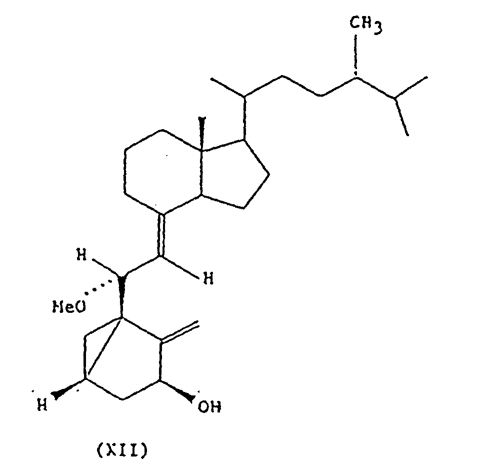

9. A prophylactic or therapeutic pharmaceutical composition, comprising an amount of a compound of the formula (I'): 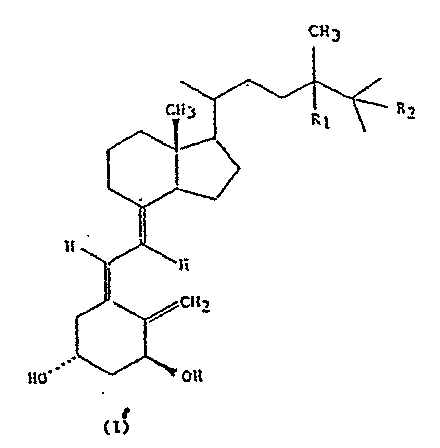 wherein R1 is either H or OH and R2 is either H or OH in combination with a pharmaceutically acceptable vehicle.

10. A pharmaceutical composition according to claim 9 wherein the amount of the compound of the formula (I') is effective to
increase serum calcium in a patient suffering vitamin D deficiency and/or
control calcium metabolism and/or
treat abnormal calcium metabolism in a mammal suffering from vitamin D deficiency, and/or
treat osteoporosis.

11. A pharmaceutical composition according to claim 9 or claim 10 wherein the amount of the compound of the formula (I') is suitable to be administered orally.

12. A method of preparing 1α-hydroxy vitamin D₄, comprising:
(a) tosylating vitamin D₄ to form vitamin D₄ tosylate;
(b) solvolyzing the vitamin D₄ tosylate to form 3,5 cyclovitamin D₄;
(c) oxidizing the 3,5 cyclovitamin D₄ to form 1α-hydroxy 3,5 cyclovitamin D₄; and
(d) sequentially solvolyzing and subjecting to a Diels-Alder reaction the 1α-hydroxy-3,5 cyclovitamin D₄ to form 1α-hydroxy vitamin D₄.

13. A method of producing vitamin D₄ tosylate, comprising reacting vitamin D₄ with toluenesulfonyl chloride in the presence of dry pyridine.

14. A method of producing 3,5 cyclovitamin D₄, comprising subjecting vitamin D₄ tosylate to buffered solvolysis.

15. A method of producing lα-hydroxy 3,5 cyclovitamin D₄, comprising allylically oxidizing the 3,5 cyclovitamin D₄ with selenium dioxide.

16. A method of producing lα-hydroxy vitamin D₄, comprising solvolizing the 1α-hydroxy 3,5 cyclovitamin D₄ with a mixture of dimethylsulfoxide and an organic acid to form an admixture of the 5,6 cis 1α-hydroxy and 5,6 trans 1α-hydroxy vitamin D₄ and subjecting the admixture to a Diels-Alder reaction forming an adduct of the 5,6 trans 1α-hydroxy vitamin D₄ to yield the 1α-hydroxy vitamin D₄.

17. A method of producing 1α-hydroxy vitamin D₄, comprising:
reducing ergosterol to 22,23 dihydroergosterol, irradiating the 22,23 dihydroergosterol to form vitamin D₄, and hydroxylating vitamin D₄ to form 1α-hydroxy vitamin D₄.

18. A method of producing 1α-hydroxy vitamin D₄, comprising:
(a) acetylating ergosterol to form ergosteryl acetate;
(b) hydroxyhalogenating the ergosteryl acetate to form 3β-acetoxy-6α-chloroergosta-7,22-dien-5α-ol;
(c) reducing and reacylating the 3β-acetoxy-6α-ergosta-7,22-dien-5α-ol to 3β-acetoxyergosta-7,22-dien-5α-ol;
(d) hydrogenating the 3β-acetoxyergosta-7,22-dien-5α-ol to form 3β-acetoxyergsto-7-en-5α-ol;
(e) reducing the 3β-acetoxyergsto-7-en-5α-ol to form 22,23 dihydroergosteryl acetate;
(f) reducing the 22,23 dihydroergosteryl acetate to 22, 23 dihydroergosterol;
(g) irradiating 22,23 dihydroergosterol to form vitamin D₄;
(h) tosylating vitamin D₄ in the presence of dry pyridine to form vitamin D₄ tosylate;
(i) solvolyzing vitamin D₄ tosylate to form 3,5 cyclovitamin D₄;
(j) allylically oxidizing the 3,5 cyclovitamin D₄ with selenium dioxide to form 1α-hydroxy vitamin D₄; and
(k) solvolyzing the 1α-hydroxy 3,5 cyclovitamin D₄ with a mixture of dimethylsulfoxide and an organic acid to form an admixture of the 5,6 cis 1α-hydroxy and 5,6 trans 1α-hydroxy vitamin D₄ and forming a Diels-Alder adduct of the 5,6 trans 1α-hydroxy vitamin D₄ to yield 1α-hydroxy vitamin D₄.

19. A feed for mammals comprising at least one compound of the formula (I') as set out in claim 9 wherein R1 is either H or OH and R2 is either H or OH wherein normal consumption of the feed by the mammals provides about 0.01 to about 0.5 µg/kg/day of said compound.

20. Use of a compound of formula (I') as set out in claim 9 (for the manufacture of a medicament for the treatment of vitamin D deficiency induced disease,
and/or osteoporosis,
and/or hyperproliferative skin disorders
and/or hypocalcemia,
and/or to control calcium metabolism in a mammal.

21. Use according to claim 20 wherein the medicament is for oral, intramuscular or intravenous administration.

22. Use according to claim 20 or claim 21 wherein the medicament is administered by daily dosage of 0.04 µg to 1.5 µg per kg of body weight of the treated mammal.

23. Use according to claim 20, claim 21 or claim 22 wherein the hypocalcemia is vitamin D dependent rickets, post operative hypoparathyroidism, renal osteodystrophy, liver cirrhosis or steatorrhoea.

## Claims (Claims for the following Contracting State(s): ES)

1. The compound of the formula (I): 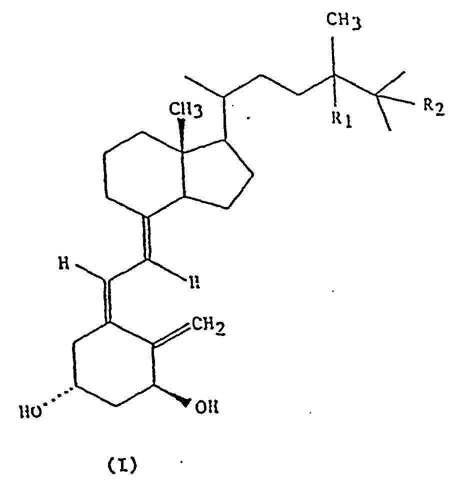 wherein R₁ is either H or OH and R₂ is either H or OH and salts, hydrates and solvates thereof and wherein the compound is not 1,25 dihydroxy vitamin D₄.

2. The compound of claim 1, wherein said compound is 1α-hydroxy vitamin D₄.

3. The compound of claim 1, wherein said compound is 1,24 dihydroxy vitamin D₄.

4. The compound of claim 1, wherein said compound is biologically active.

5. The compound of any one of the preceding claims wherein said compound exhibits biological activity approaching that of 1,25 vitamin D₃ and wherein said compound is less toxic than 1α-hydroxy vitamin D₃ as determined by comparative LD₅₀ values in rats.

6. The vitamin D₄ tosylate compound of the formula (X) :

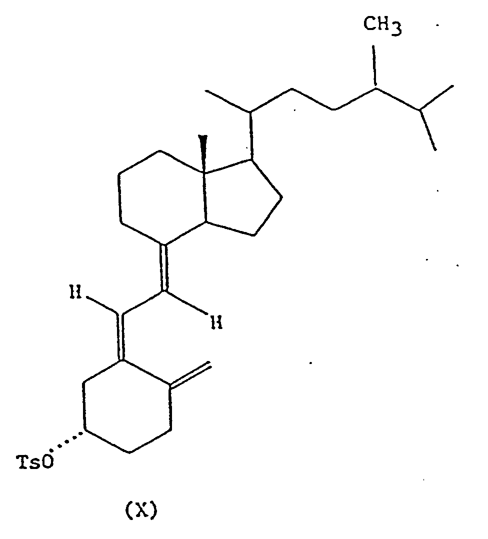

7. The 3,5 cyclovitamin D₄ compound of the formula (XI) :

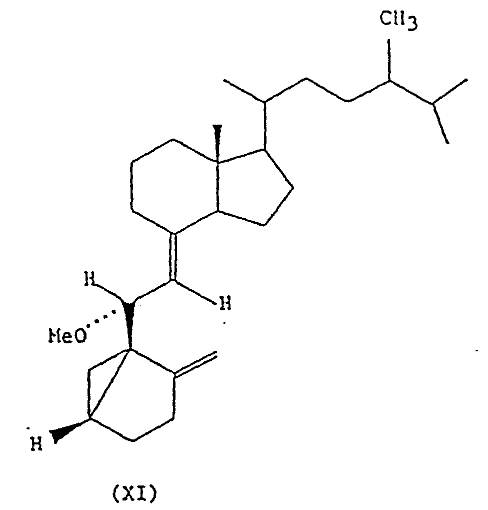

8. The 1α-hydroxy 3,5 cyclovitamin D₄ of the formula (XII):

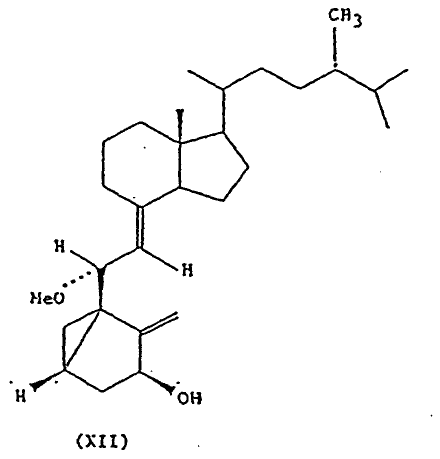

9. A prophylactic or therapeutic pharmaceutical composition, comprising an amount of a compound of the formula (I'): 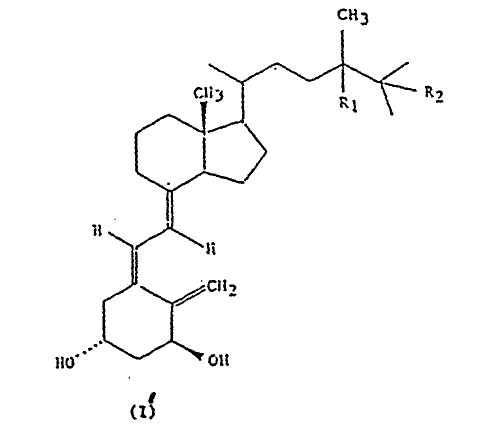 wherein R1 is either H or OH and R2 is either H or OH in combination with a pharmaceutically acceptable vehicle.

10. A pharmaceutical composition according to claim 9 wherein the amount of the compound of the formula (I') is effective to
increase serum calcium in a patient suffering vitamin D deficiency and/or
control calcium metabolism and/or
treat abnormal calcium metabolism in a mammal suffering from vitamin D deficiency, and/or
treat osteoporosis.

11. A pharmaceutical composition according to claim 9 or claim 10 wherein the amount of the compound of the formula (I') is suitable to be administered orally.

12. A method of preparing 1α-hydroxy vitamin D₄, comprising:
(a) tosylating vitamin D₄ to form vitamin D₄ tosylate;
(b) solvolyzing the vitamin D₄ tosylate to form 3,5 cyclovitamin D₄;
(c) oxidizing the 3,5 cyclovitamin D₄ to form 1α-hydroxy 3,5 cyclovitamin D₄; and
(d) sequentially solvolyzing and subjecting to a Diels-Alder reaction the 1α-hydroxy-3,5 cyclovitamin D₄ to form 1α-hydroxy vitamin D₄.

13. A method of producing vitamin D₄ tosylate, comprising reacting vitamin D₄ with toluenesulfonyl chloride in the presence of dry pyridine.

14. A method of producing 3,5 cyclovitamin D₄, comprising subjecting vitamin D₄ tosylate to buffered solvolysis.

15. A method of producing 1α-hydroxy 3,5 cyclovitamin D₄, comprising allylically oxidizing the 3,5 cyclovitamin D₄ with selenium dioxide.

16. A method of producing 1α-hydroxy vitamin D₄, comprising solvolizing the 1α-hydroxy 3,5 cyclovitamin D₄ with a mixture of dimethylsulfoxide and an organic acid to form an admixture of the 5,6 cis 1α-hydroxy and 5,6 trans 1α-hydroxy vitamin D₄ and subjecting the admixture to a Diels-Alder reaction forming an adduct of the 5,6 trans 1α-hydroxy vitamin D₄ to yield the 1α-hydroxy vitamin D₄.

17. A method of producing 1α-hydroxy vitamin D₄, comprising:
reducing ergosterol to 22,23 dihydroergosterol, irradiating the 22,23 dihydroergosterol to form vitamin D₄, and hydroxylating vitamin D₄ to form 1α-hydroxy vitamin D₄.

18. A method of producing 1α-hydroxy vitamin D₄, comprising:
(a) acetylating ergosterol to form ergosteryl acetate;
(b) hydroxyhalogenating the ergosteryl acetate to form 3β-acetoxy-6α-chloroergosta-7,22-dien-5α-ol;
(c) reducing and reacylating the 3β-acetoxy-6α-ergosta-7,22-dien-5α-ol to 3β-acetoxyergosta-7,22-dien-5α-ol;
(d) hydrogenating the 3β-acetoxyergosta-7,22-dien-5α-ol to form 3β-acetoxyergsto-7-en-5α-ol;
(e) reducing the 3β-acetoxyergsto-7-en-5α-ol to form 22,23 dihydroergosteryl acetate;
(f) reducing the 22,23 dihydroergosteryl acetate to 22, 23 dihydroergosterol;
(g) irradiating 22,23 dihydroergosterol to form vitamin D₄;
(h) tosylating vitamin D₄ in the presence of dry pyridine to form vitamin D₄ tosylate;
(i) solvolyzing vitamin D₄ tosylate to form 3,5 cyclovitamin D₄;
(j) allylically oxidizing the 3,5 cyclovitamin D₄ with selenium dioxide to form 1α-hydroxy vitamin D₄; and
(k) solvolyzing the 1α-hydroxy 3,5 cyclovitamin D₄ with a mixture of dimethylsulfoxide and an organic acid to form an admixture of the 5,6 cis 1α-hydroxy and 5,6 trans 1α-hydroxy vitamin D₄ and forming a Diels-Alder adduct of the 5,6 trans 1α-hydroxy vitamin D₄ to yield 1α-hydroxy vitamin D₄.

19. A feed for mammals comprising at least one compound of the formula (I') as set out in claim 9 wherein R1 is either H or OH and R2 is either H or OH wherein normal consumption of the feed by the mammals provides about 0.01 to about 0.5 µg/kg/day of said compound.

20. Use of a compound of formula (I') as set out in claim 9 for the manufacture of a medicament for the treatment of vitamin D deficiency induced disease,
and/or osteoporosis,
and/or hyperproliferative skin disorders and/or hypocalcemia,
and/or to control calcium metabolism in a mammal.

21. Use according to claim 20 wherein the medicament is for oral, intramuscular or intravenous administration.

22. Use according to claim 20 or claim 21 wherein the medicament is administered by daily dosage of 0.04 µg to 1.5 µg per kg of body weight of the treated mammal.

23. Use according to claim 20, claim 21 or claim 22 wherein the hypocalcemia is vitamin D dependent rickets, post operative hypoparathyroidism, renal osteodystrophy, liver cirrhosis or steatorrhoea.

24. A method for producing 1,24 dihydroxy vitamin D₄ comprising incubating 1α-hydroxy vitamin D₄ with human liver cells, culturing the cells, and recovering 1,24 dihydroxy vitamin D₄.

25. A method of preparing a prophylactic or therapeutic pharmaceutical composition comprising mixing an amount of a compound of the formula (I') with a pharmaceutically acceptable carrier substance suitable for enteral, parenteral or topical application: 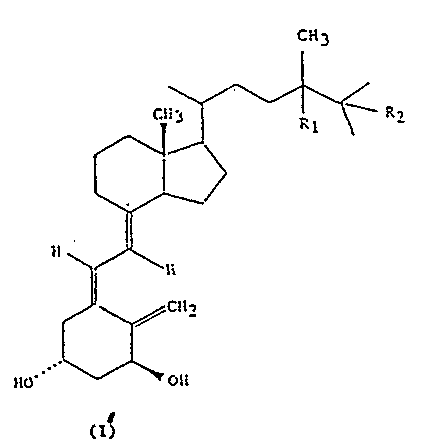 wherein R1 is either H or OH and R2 is either H or OH in combination with a pharmaceutically acceptable vehicle.

## Patentansprüche (Patentansprüche für folgende(n) Vertragsstaat(en): DE, DK, FR, GB, IT, NL)

1. Verbindung der Formel (I): 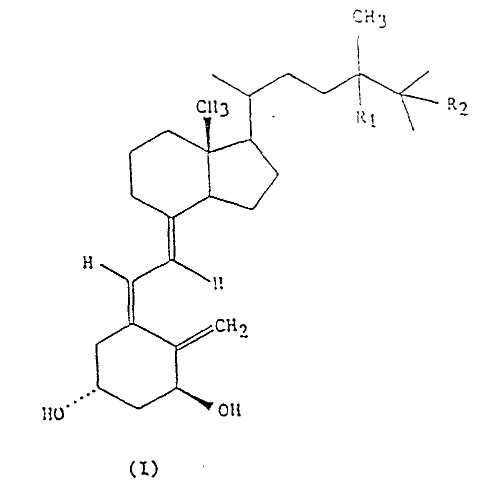 worin R₁ entweder H oder OH ist und R₂ entweder H oder OH ist, sowie Salze, Hydrate und Solvate davon, wobei die Verbindung nicht 1,25-Dihydroxy-Vitamin D₄ ist.

2. Verbindung nach Anspruch 1, worin die Verbindung 1α-Hydroxy-Vitamin D₄ ist.

3. Verbindung nach Anspruch 1, worin die Verbindung 1,24-Dihydroxy-Vitamin D₄ ist.

4. Verbindung nach Anspruch 1, worin die Verbindung biologisch aktiv ist.

5. Verbindung nach einem der vorangegangenen Ansprüche, worin die Verbindung biologische Aktivität aufweist, die der von 1,25-Vitamin D₃ nahe kommt, und worin die Verbindung weniger toxisch als 1α-Hydroxy-Vitamin D₃ ist, wie durch LD₅₀-Vergleichswerte bei Ratten ermittelt.

6. Vitamin D₄-tosylat-Verbindung der Formel (X):

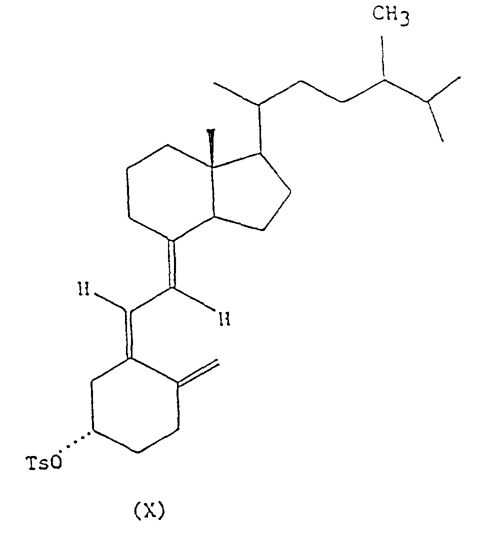

7. 3,5-Cyclovitamin D₄-Verbindung der Formel (XI):

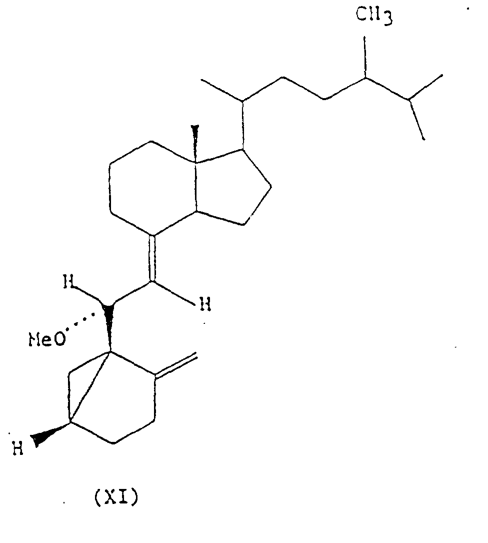

8. 1α-Hydroxy-3,5-cyclovitamin D₄ der Formel (XII):

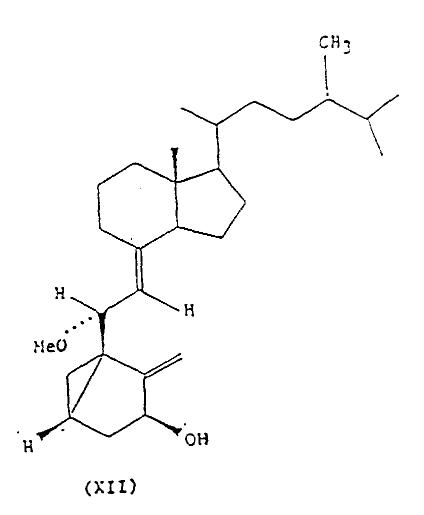

9. Prophylaktische oder therapeutische pharmazeutische Zusammensetzung, die eine bestimmte Menge einer Verbindung der Formel (I'): 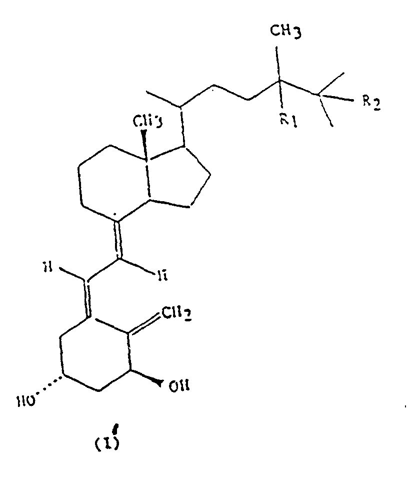 worin R1 entweder H oder OH ist und R2 entweder H oder OH ist, in Kombination mit einem pharmazeutisch annehmbaren Vehikel umfasst.

10. Pharmazeutische Zusammensetzung nach Anspruch 9, worin die Menge der Verbindung der Formel (I') dazu dient,
den Kalziumspiegel im Serum eines Patienten, der an Vitamin D-Mangel leidet, zu erhöhen, und/oder
den Kalzium-Stoffwechsel zu regulieren, und/oder
Kalzium-Stoffwechselstörungen bei einem Säugetier, das an Vitamin D-Mangel leidet, zu behandeln, und/oder
Osteoporose zu behandeln.

11. Pharmazeutische Zusammensetzung nach Anspruch 9 oder 10, worin die Menge der Verbindung der Formel (I') zur oralen Verabreichung geeignet ist.

12. Verfahren zur Herstellung von 1α-Hydroxy-Vitamin D₄, umfassend:
(a) das Tosylieren von Vitamin D₄, um Vitamin D₄-tosylat zu bilden;
(b) das Solvolysieren des Vitamin D₄-tosylats, um 3,5-Cyclovitamin D₄ zu bilden;
(c) das Oxidieren des 3,5-Cyclovitamins D₄, um 1α-Hydroxy-3,5-cyclovitamin D₄ zu bilden; und
(d) das aufeinander folgende Solvolysieren des 1α-Hydroxy-3,5-cyclovitamins D₄ und Unterziehen desselben einer Diels-Alder-Reaktion, um 1α-Hydroxy-Vitamin D₄ zu bilden.

13. Verfahren zur Herstellung von Vitamin D₄-tosylat, umfassend das Umsetzen von Vitamin D₄ mit Toluolsulfonylchlorid in Gegenwart von trockenem Pyridin.

14. Verfahren zur Herstellung von 3,5-Cyclovitamin D₄, umfassend die Durchführung von gepufferter Solvolyse von Vitamin D₄-tosylat.

15. Verfahren zur Herstellung von 1α-Hydroxy-3,5-cyclovitamin D₄, umfassend das allylische Oxidieren des 3,5-Cyclovitamins D₄ mit Selendioxid.

16. Verfahren zur Herstellung von 1α-Hydroxy-Vitamin D₄, umfassend das Solvolysieren des 1α-Hydroxy-Vitamins D₄ mit einem Gemisch aus Dimethylsulfoxid und einer organischen Säure, um ein Gemisch aus 5,6-cis-1α-Hydroxy- und 5,6-trans-1α-Hydroxy-Vitamin D₄ zu bilden, sowie das Unterziehen des Gemisches einer Diels-Alder-Reaktion, wodurch ein Addukt von 5,6-trans-1α-Hydroxy-Vitamin D₄ gebildet wird, was 1α-Hydroxy-Vitamin D₄ ergibt.

17. Verfahren zur Herstellung von 1α-Hydroxy-Vitamin D₄, umfassend:
das Reduzieren von Ergosterol zu 22,23-Dihydroergosterol,
das Bestrahlen von 22,23-Dihydroergosterol, um Vitamin D₄ zu bilden, und
das Hydroxylieren von Vitamin D₄, um 1α-Hydroxy-Vitamin D₄ zu bilden.

18. Verfahren zur Herstellung von 1α-Hydroxy-Vitamin D₄, umfassend:
(a) das Acetylieren von Ergosterol, um Ergosterylacetat zu bilden;
(b) das Hydroxyhalogenieren des Ergosterylacetats, um 3β-Acetoxy-6α-chlorergosta-7,22-dien-5α-ol zu bilden;
(c) das Reduzieren und Reacylieren des 3β-Acetoxy-6α-chlorergosta-7,22-dien-5α-ols zu 3ß-Acetoxyergosta-7,22-dien-5α-ol;
(d) das Hydrieren des 3ß-Acetoxyergosta-7,22-dien-5α-ols, um 3β-Acetoxyergost-7-en-5α-ol zu bilden;
(e) das Reduzieren des 3ß-Acetoxyergost-7-en-5α-ols, um 22,23-Dihydroergosterylacetat zu bilden;
(f) das Reduzieren des 22,23-Dihydroergosterylacetats zu 22,23-dihydroergosterol;
(g) das Bestrahlen von 22,23-Dihydroergosterol, um Vitamin D₄ zu bilden;
(h) das Tosylieren von Vitamin D₄ in Gegenwart von trockenem Pyridin, um Vitamin D₄-tosylat zu bilden;
(i) das Solvolysieren von Vitamin D₄-tosylat, um 3,5-Cyclovitamin D₄ zu bilden;
(j) das allylische Oxidieren des 3,5-Cyclovitamins D₄ mit Selendioxid, um 1α-Hydroxyvitamin D₄ zu bilden; und
(k) das Solvolysieren des 1α-Hydroxy-3,5-cyclovitamins D₄ mit einem Gemisch aus Dimethylsulfoxid und einer organischen Säure, um ein Gemisch aus 5,6-cis-1α-Hydroxy- und 5,6-trans-1α-Hydroxy-Vitamin D₄ zu bilden, sowie das Bilden eines Diels-Alder-Adduktes des 5,6-trans-1α-Hydroxy-Vitamins D₄, was 1α-Hydroxy-Vitamin D₄ ergibt.

19. Nahrungsmittel für Säugetiere, das zumindest eine Verbindung der Formel (I'), wie in Anspruch 9 dargelegt, umfasst, worin R1 entweder H oder OH ist und R2 entweder H oder OH ist, worin normaler Verzehr des Nahrungsmittels durch die Säugetiere für etwa 0,01 bis etwa 0,5 µg/kg/Tag der Verbindung sorgt.

20. Verwendung einer Verbindung der Formel (I'), wie in Anspruch 9 dargelegt, zur Herstellung eines Medikaments zur Behandlung von durch Vitamin D-Mangel verursachten Erkrankungen; und/oder
Osteoporose; und/oder
hyperproliferativen Hauterkrankungen; und/oder
Hypokalzämie; und/oder
zur Regulierung des Kalzium-Stoffwechsels bei einem Säugetier.

21. Verwendung nach Anspruch 20, worin das Medikament zur oralen, intramuskulären oder intravenösen Verabreichung dient.

22. Verwendung nach Anspruch 20 oder 21, worin das Medikament in täglichen Dosen von 0,04 µg bis 1,5 µg pro kg Körpergewicht des behandelten Säugetiers verabreicht wird.

23. Verwendung nach Anspruch 20, 21 oder 22, worin die Hypokalzämie Vitamin-D-abhängige Rachitis, postoperativer Hypoparathyreoidismus, renale Osteodystrophie, Leberzirrhose oder Steatorrhoe ist.

## Patentansprüche (Patentansprüche für folgende(n) Vertragsstaat(en): ES)

1. Verbindung der Formel (I): 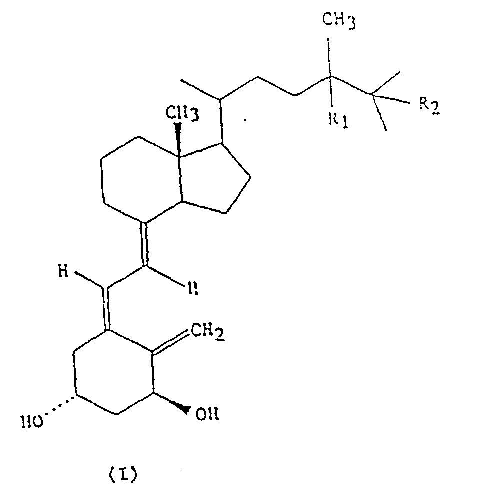 worin R₁ entweder H oder OH ist und R₂ entweder H oder OH ist, sowie Salze, Hydrate und Solvate davon, wobei die Verbindung nicht 1,25-Dihydroxy-Vitamin D₄ ist.

2. Verbindung nach Anspruch 1, worin die Verbindung 1α-Hydroxy-Vitamin D₄ ist.

3. Verbindung nach Anspruch 1, worin die Verbindung 1,24-Dihydroxy-Vitamin D₄ ist.

4. Verbindung nach Anspruch 1, worin die Verbindung biologisch aktiv ist.

5. Verbindung nach einem der vorangegangenen Ansprüche, worin die Verbindung biologische Aktivität aufweist, die der von 1,25-Vitamin D₃ nahe kommt, und worin die Verbindung weniger toxisch als 1α-Hydroxy-Vitamin D₃ ist, wie durch LD₅₀-Vergleichswerte bei Ratten ermittelt.

6. Vitamin D₄-tosylat-Verbindung der Formel (X):

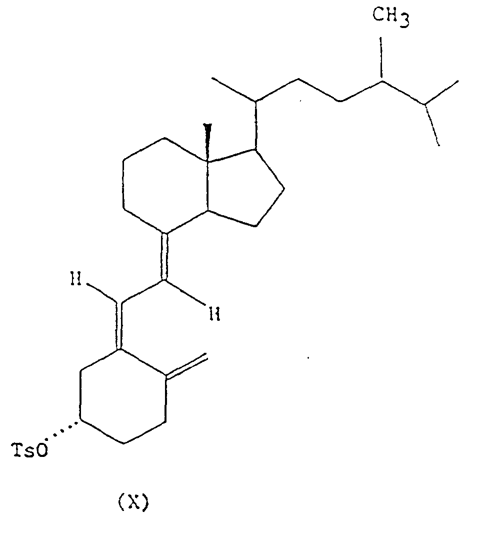

7. 3,5-Cyclovitamin D₄-Verbindung der Formel (XI):

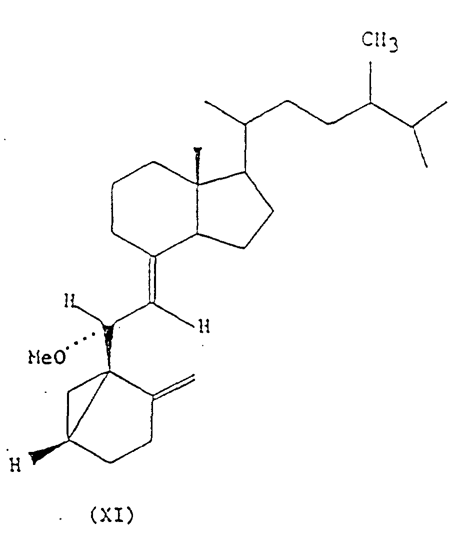

8. 1α-Hydroxy-3,5-cyclovitamin D₄ der Formel (XII):

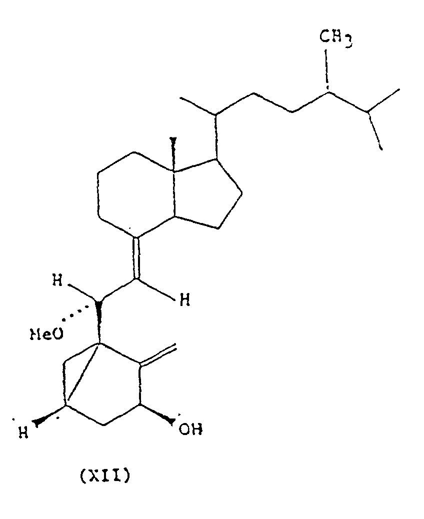

9. Prophylaktische oder therapeutische pharmazeutische Zusammensetzung, die eine bestimmte Menge einer Verbindung der Formel (I'): 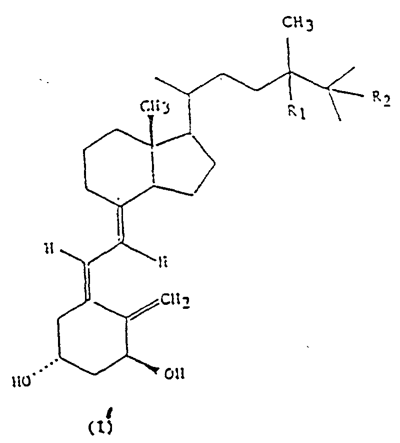 worin R1 entweder H oder OH ist und R2 entweder H oder OH ist, in Kombination mit einem pharmazeutisch annehmbaren Vehikel umfasst.

10. Pharmazeutische Zusammensetzung nach Anspruch 9, worin die Menge der Verbindung der Formel (I') dazu dient,
den Kalziumspiegel im Serum eines Patienten, der an Vitamin D-Mangel leidet, zu erhöhen, und/oder
den Kalzium-Stoffwechsel zu regulieren, und/oder
Kalzium-Stoffwechselstörungen bei einem Säugetier, das an Vitamin D-Mangel leidet, zu behandeln, und/oder
Osteoporose zu behandeln.

11. Pharmazeutische Zusammensetzung nach Anspruch 9 oder 10, worin die Menge der Verbindung der Formel (I') zur oralen Verabreichung geeignet ist.

12. Verfahren zur Herstellung von 1α-Hydroxy-Vitamin D₄, umfassend:
(a) das Tosylieren von Vitamin D₄, um Vitamin D₄-tosylat zu bilden;
(b) das Solvolysieren des Vitamin D₄-tosylats, um 3,5-Cyclovitamin D₄ zu bilden;
(c) das Oxidieren des 3,5-Cyclovitamins D₄, um 1α-Hydroxy-3,5-cyclovitamin D₄ zu bilden; und
(d) das aufeinander folgende Solvolysieren des 1α-Hydroxy-3,5-cyclovitamins D₄ und Unterziehen desselben einer Diels-Alder-Reaktion, um 1α-Hydroxy-Vitamin D₄ zu bilden.

13. Verfahren zur Herstellung von Vitamin D₄-tosylat, umfassend das Umsetzen von Vitamin D₄ mit Toluolsulfonylchlorid in Gegenwart von trockenem Pyridin.

14. Verfahren zur Herstellung von 3,5-Cyclovitamin D₄, umfassend die Durchführung von gepufferter Solvolyse von Vitamin D₄-tosylat.

15. Verfahren zur Herstellung von 1α-Hydroxy-3,5-cyclovitamin D₄, umfassend das allylische Oxidieren des 3,5-Cyclovitamins D₄ mit Selendioxid.

16. Verfahren zur Herstellung von 1α-Hydroxy-Vitamin D₄, umfassend das Solvolysieren des 1α-Hydroxy-Vitamins D₄ mit einem Gemisch aus Dimethylsulfoxid und einer organischen Säure, um ein Gemisch aus 5,6-cis-1α-Hydroxy- und 5,6-trans-1α-Hydroxy-Vitamin D zu bilden, sowie das Unterziehen des Gemisches einer Diels-Alder-Reaktion, wodurch ein Addukt von 5,6-trans-1α-Hydroxy-Vitamin D₄ gebildet wird, was 1α-Hydroxy-Vitamin D₄ ergibt.

17. Verfahren zur Herstellung von 1α-Hydroxy-Vitamin D₄, umfassend:
das Reduzieren von Ergosterol zu 22,23-Dihydroergosterol,
das Bestrahlen von 22,23-Dihydroergosterol, um Vitamin D₄ zu bilden, und
das Hydroxylieren von Vitamin D₄, um 1α-Hydroxy-Vitamin D₄ zu bilden.

18. Verfahren zur Herstellung von 1α-Hydroxy-Vitamin D₄, umfassend:
(a) das Acetylieren von Ergosterol, um Ergosterylacetat zu bilden;
(b) das Hydroxyhalogenieren des Ergosterylacetats, um 3β-Acetoxy-6α-chlorergosta-7,22-dien-5α-ol zu bilden;
(c) das Reduzieren und Reacylieren des 3β-Acetoxy-6α-chlorergosta-7,22-dien-5α-ols zu 3β-Acetoxyergosta-7,22-dien-5α-ol;
(d) das Hydrieren des 3β-Acetoxyergosta-7,22-dien-5α-ols, um 3β-Acetoxyergost-7-en-5α-ol zu bilden;
(e) das Reduzieren des 3β-Acetoxyergost-7-en-5α-ols, um 22,23-Dihydroergosterylacetat zu bilden;
(f) das Reduzieren des 22,23-Dihydroergosterylacetats zu 22,23-dihydroergosterol;
(g) das Bestrahlen von 22,23-Dihydroergosterol, um Vitamin D₄ zu bilden;
(h) das Tosylieren von Vitamin D₄ in Gegenwart von trockenem Pyridin, um Vitamin D₄-tosylat zu bilden;
(i) das Solvolysieren von Vitamin D₄-tosylat, um 3,5-Cyclovitamin D₄ zu bilden;
(j) das allylische Oxidieren des 3,5-Cyclovitamins D₄ mit Selendioxid, um 1α-Hydroxyvitamin D₄ zu bilden; und
(k) das Solvolysieren des 1α-Hydroxy-3,5-cyclovitamins D₄ mit einem Gemisch aus Dimethylsulfoxid und einer organischen Säure, um ein Gemisch aus 5,6-cis-1α-Hydroxy- und 5,6-trans-1α-Hydroxy-Vitamin D₄ zu bilden, sowie das Bilden eines Diels-Alder-Adduktes des 5,6-trans-1α-Hydroxy-Vitamins D₄, was 1α-Hydroxy-Vitamin D₄ ergibt.

19. Nahrungsmittel für Säugetiere, das zumindest eine Verbindung der Formel (I'), wie in Anspruch 9 dargelegt, umfasst, worin R1 entweder H oder OH ist und R2 entweder H oder OH ist, worin normaler Verzehr des Nahrungsmittels durch die Säugetiere für etwa 0,01 bis etwa 0,5 µg/kg/Tag der Verbindung sorgt.

20. Verwendung einer Verbindung der Formel (I'), wie in Anspruch 9 dargelegt, zur Herstellung eines Medikaments zur Behandlung von durch Vitamin D-Mangel verursachten Erkrankungen; und/oder
Osteoporose; und/oder
hyperproliferativen Hauterkrankungen; und/oder
Hypokalzämie; und/oder
zur Regulierung des Kalzium-Stoffwechsels bei einem Säugetier.

21. Verwendung nach Anspruch 20, worin das Medikament zur oralen, intramuskulären oder intravenösen Verabreichung dient.

22. Verwendung nach Anspruch 20 oder 21, worin das Medikament in täglichen Dosen von 0,04 µg bis 1,5 µg pro kg Körpergewicht des behandelten Säugetiers verabreicht wird.

23. Verwendung nach Anspruch 20, 21 oder 22, worin die Hypokalzämie Vitamin-D-abhängige Rachitis, postoperativer Hypoparathyreoidismus, renale Osteodystrophie, Leberzirrhose oder Steatorrhoe ist.

24. Verfahren zur Herstellung von 1,24-Dihydroxy-Vitamin D₄, umfassend das lnkubieren von 1α-Hydroxy-Vitamin D₄ mit menschlichen Leberzellen, das Kultivieren der Zellen und das Gewinnen von 1,24-Dihydroxy-Vitamin D₄.

25. Verfahren zur Herstellung einer prophylaktischen oder therapeutischen pharmazeutischen Zusammensetzung, umfassend das Vermischen einer betimmten Menge einer Verbindung der Formel (I') mit einer pharmazeutisch annehmbaren Trägersubstanz, die zur enteralen, parenteralen oder topischen Anwendung geeignet ist: 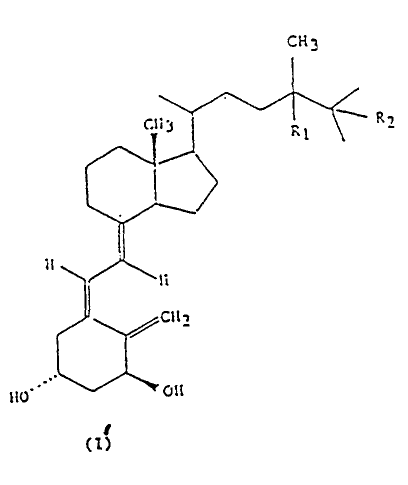 worin R1 entweder H oder OH ist und R2 entweder H oder OH ist, in Kombination mit einem pharmazeutisch annehmbaren Vehikel.

## Revendications (Revendications pour l'(les) Etat(s) contractant(s) suivant(s): DE, DK, FR, GB, IT, NL)

1. Composé de la formule (I) : 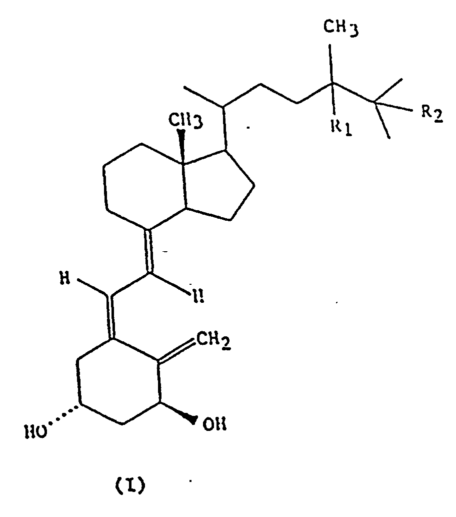 où R₁ st H ou OH et R₂ est H ou OH et des sels, hydrates et solvates de ceux-ci et où le composé n'est pas 1,25 dihydroxy vitamine D₄.

2. Composé de la revendication 1, où ledit composé est la 1α-hydroxy vitamine D₄.

3. Composé de la revendication 1, où ledit composé est la 1,24 dihydroxy vitamine D₄.

4. Composé de la revendication 1, où ledit composé est biologiquement actif.

5. Composé selon l'une quelconque des revendications précédentes, où ledit composé présente une activité biologique s'approchant de celle de la 1,25 vitamine D₄ et où ledit composé est moins toxique que le 1α-hydroxy vitamine D3 en déterminant par les valeurs de LD₅₀ de comparaison chez les rats.

6. Composé de tosylate de la vitamine D₄ de la formule (X) :

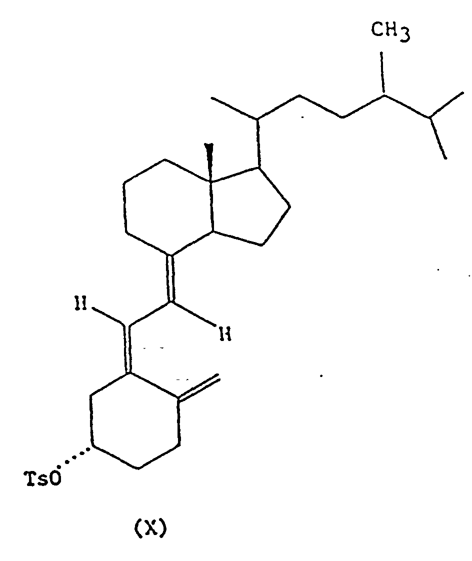

7. Composé de 3,5 cyclovitamine D₄ de la formule (XI) :

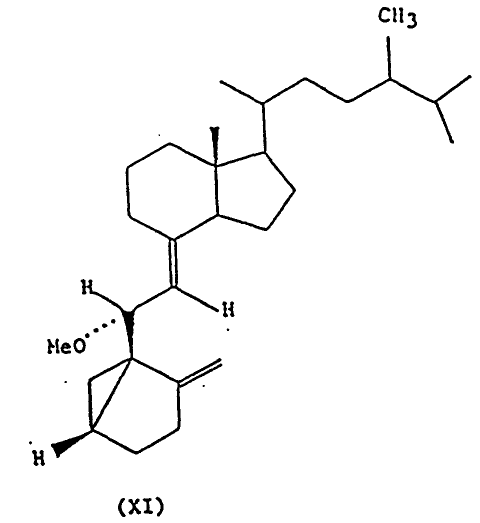

8. 1α-hydroxy 3,5 cyclovitamine D₄ de la formule (XII) :

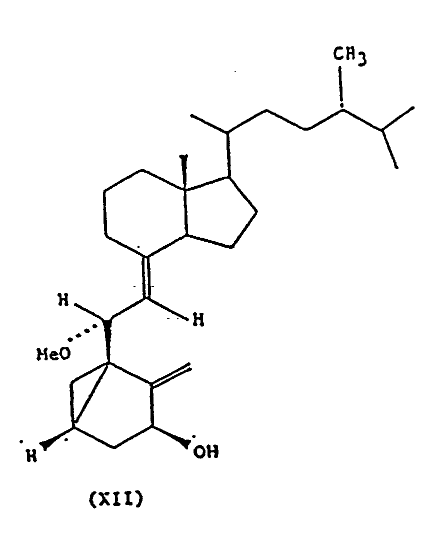

9. Composition pharmaceutique prophylactique ou thérapeutique comprenant une quantité d'un composé de la formule (I') : 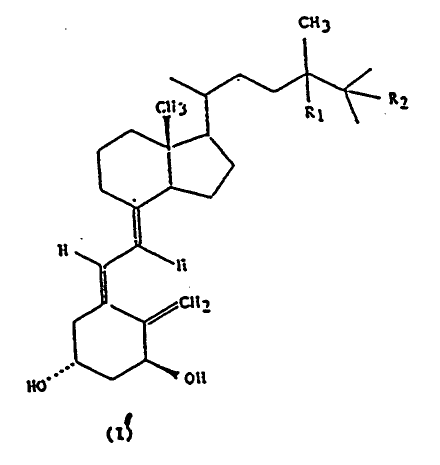 où R₁ est H ou OH et R₂ est H ou OH en combinaison avec un véhicule pharmaceutiquement acceptable.

10. Composition pharmaceutique selon la revendication 9, où la quantité du composé de la formule (I') est efficace pour
augmenter le calcium dans le sérum chez un patient souffrant d'une déficience en vitamine D et/ou
contrôler le métabolisme du calcium et/ou
traiter un métabolisme anormal du calcium chez un mammifère souffrant d'une déficience en vitamine D, et/ou
traiter l'ostéoporose.

11. Composition pharmaceutique selon la revendication 9 ou la revendication 10, où la quantité du composé de la formule (I') est appropriée pour être administrée oralement.

12. Méthode de préparation de 1α-hydroxy vitamine D4 consistant à :
a) tosyler la vitamine D₄ pour former le tosylate de vitamine D4 ;
b) solvolyser le tosylate de la vitamine D₄ pour former la 3,5 cyclovitamine D₄ ;
c) oxyder la 3,5 cyclovitamine D₄ pour former la 1α-hydroxy 3,5 cyclovitamine D₄ ; et
d) séquentiellement solvolyser et soumettre à une réaction de Diels-Alder la 1α-hydroxy 3,5 cyclovitamine D₄ pour former la 1α-hydroxy vitamine D₄.

13. Méthode de production du tosylate de la vitamine D₄ comprenant la réaction de la vitamine D₄ avec du chlorure de toluènesulfonyle en présence de pyridine sèche.

14. Méthode de production de 3,5 cyclovitamine D₄ consistant à soumettre le tosylate de la vitamine D₄ à une solvolyse tamponnée.

15. Méthode de production de 1α-hydroxy 3,5 cyclovitamine D₄, comprenant l'oxydation allylique de la 3,5 cyclovitamine D₄ avec du bioxyde de sélénium.

16. Méthode de production de 1α-hydroxy vitamine D4 comprenant la solvolyse de la 1α-hydroxy 3,5 cyclovitamine D₄ avec un mélange de diméthylsulfoxyde et d'un acide organique pour former un mélange de 5,6 cis 1α-hydroxy et 5,6 trans 1α-hydroxy vitamine D₄ et consistant à soumettre le mélange à une réaction de Diels-Alder pour former un produit d'addition de la 5,6 trans 1α-hydroxy vitamine D₄ pour donner la 1α-hydroxy vitamine D₄.

17. Méthode de production de 1α-hydroxy vitamine D₄ consistant à :
réduire de l'ergostérol en 22, 23 dihydroergostérol, irradier le 22, 23 dihydroergostérol pour former de la vitamine D₄ et hydroxyler la vitamine D₄ pour former la 1α-hydroxy vitamine D₄.

18. Méthode de production de 1α-hydroxy vitamine D₄ consistant à :
(a) acétyler de l'ergostérol pour former de l'acétate d'ergostéryle ;
(b) hydroxyhalogéner l'acétate d'ergostéryle pour former le 3β-acétoxy-6α-chloroergosta-7,22-dién-5α-ol ;
(c) réduire et réacyler le 3β-acétoxy-6α-ergosta-7,22-dién-5α-ol en 3β-acétoxyergosta-7,22-dien-5α-ol ;
(d) hydrogéner le 3β-acétoxyergosta-7,22-dién-5α-ol pour former du 3β-acétoxyergosto-7-én-5α-ol ;
(e) réduire le 3β-acétoxyergosto-7-én-5α-ol pour former le 22,23 dihydroergostéryl acétate ;
(f) réduire le 22,23 dihydroergostéryl acétate en 22,23 dihydroergostérol ;
(g) irradier le 22,23 dihydroergostérol pour former de la vitamine D₄ ;
(h) tosyler la vitamine D₄ en présence de pyridine sèche pour former de tosylate de vitamine D₄ ;
(i) solvolyser le tosylate de vitamine D₄ pour former la 3,5 cyclovitamine D₄ ;
(j) oxyder allyliquement la 3,5 cyclovitamine D₄ avec du bioxyde de sélénium pour former la 1α-hydroxy vitamine D₄ ; et
(k) solvolyser la 1α-hydroxy vitamine D₄ avec un mélange de diméthylsulfoxyde et d'un acide organique pour former un mélange de 5,6 cis 1α-hydroxy et 5,6 trans 1α-hydroxy vitamine D₄ et former un produit d'addition de Diels-Alder de la 5,6 trans 1α-hydroxy vitamine D₄ pour donner la 1α-hydroxy vitamine D₄.

19. Alimentation pour mammifère comprenant au moins un composé de la formule (I') tel qu'indiqué à la revendication 9 où R₁ est soit H ou OH et R₂ est soit H ou OH, où la consommation normale de l'alimentation par les mammifères procure environ 0,01 à environ 0,5 µg/kg/jour dudit composé.

20. Utilisation d'un composé de formule (I') tel qu'indiqué à la revendication 9 pour la fabrication d'un médicament pour le traitement d'une maladie induite par une déficience en vitamine D,
et/ou de l'ostéoporose,
et/ou de troubles hyperprolifératifs de la peau,
et/ou d'hypocalcémie,
et/ou pour contrôler le métabolisme du calcium chez un mammifère.

21. Utilisation selon la revendication 20, où le médicament est pour administration orale, intramusculaire ou intraveineuse.

22. Utilisation selon la revendication 20 ou la revendication 21, où le médicament est administré par dosage quotidien de 0,04 µg à 1,5 µg par kg de poids corporel du mammifère traité.

23. Utilisation selon la revendication 20 ou la revendication 21 ou la revendication 22, où l'hypocalcémie est le rachitisme dépendant de la vitamine D, l'hypoparathyroidie post-opératoire, l'ostéodystrophie rénale, la cirrhose du foie ou la stéatorrhée.

## Revendications (Revendications pour l'(les) Etat(s) contractant(s) suivant(s): ES)

1. Composé de la formule (I) : 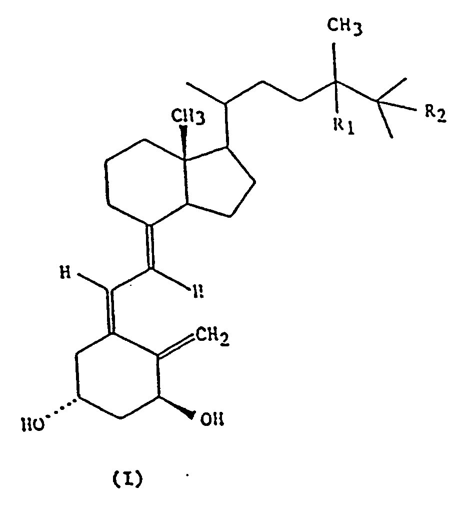 où R₁ st H ou OH et R₂ est H ou OH et des sels, hydrates et solvates de ceux-ci et où le composé n'est pas 1,25 dihydroxy vitamine D₄.

2. Composé de la revendication 1, où ledit composé est la 1α-hydroxy vitamine D₄.

3. Composé de la revendication 1, où ledit composé est la 1,24 dihydroxy vitamine D₄.

4. Composé de la revendication 1, où ledit composé est biologiquement actif.

5. Composé selon l'une quelconque des revendications précédentes, où ledit composé présente une activité biologique s'approchant de celles de la 1,25 vitamine D₄ et où ledit composé est moins toxique que le 1α-hydroxy vitamine D3 en déterminant par les valeurs de LD₅₀ de comparaison chez les rats.

6. Composé de tosylate de la vitamine D₄ de la formule (X) :

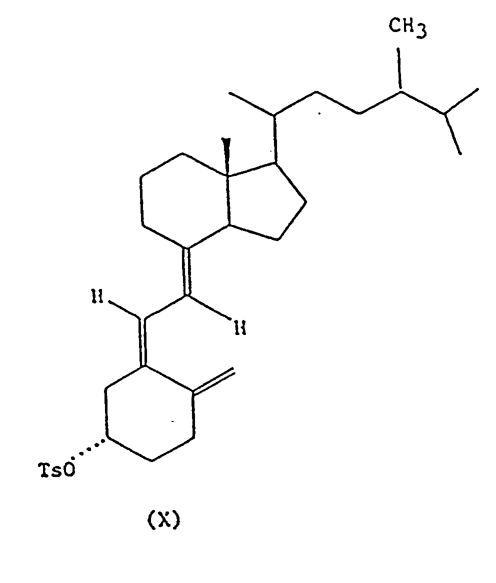

7. Composé de 3,5 cyclovitamine D₄ de la formule (XI) :

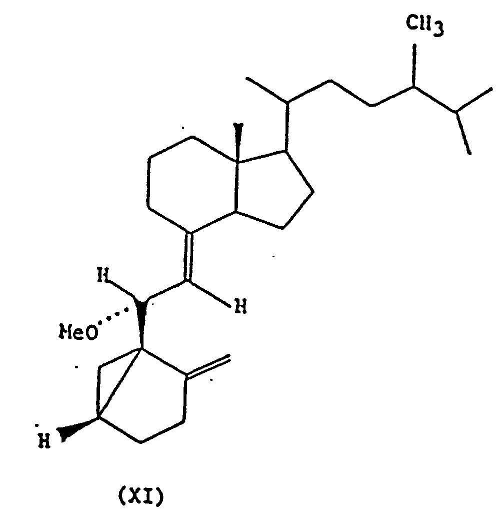

8. 1α-hydroxy 3,5 cyclovitamine D₄ de la formule (XII) :

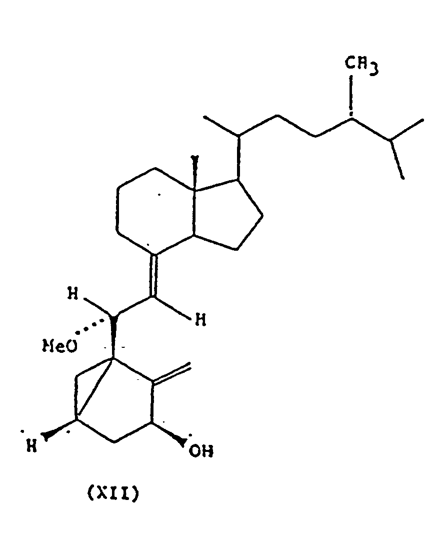

9. Composition pharmaceutique prophylactique ou thérapeutique comprenant une quantité d'un composé de la formule (I') : 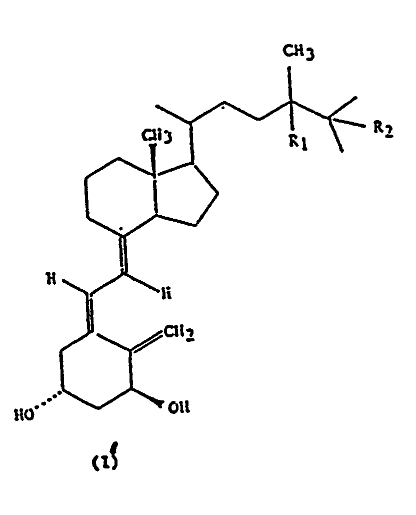 où R₁ est H ou OH et R₂ est H ou OH en combinaison avec un véhicule pharmaceutiquement acceptable.

10. Composition pharmaceutique selon la revendication 9, où la quantité du composé de la formule (I') est efficace pour
augmenter le calcium dans le sérum chez un patient souffrant d'une déficience en vitamine D et/ou
contrôler le métabolisme du calcium et/ou
traiter un métabolisme anormal du calcium chez un mammifère souffrant d'une déficience en vitamine D, et/ou
traiter l'ostéoporose.

11. Composition pharmaceutique selon la revendication 9 ou la revendication 10, où la quantité du composé de la formule (I') est appropriée pour être administrée oralement.

12. Méthode de préparation de 1α-hydroxy vitamine D4 consistant à :
a) tosyler la vitamine D₄ pour former le tosylate de vitamine D₄ ;
b) solvolyser le tosylate de la vitamine D₄ pour former la 3,5 cyclovitamine D₄ ;
c) oxyder la 3,5 cyclovitamine D₄ pour former la 1α-hydroxy 3,5 cyclovitamine D₄ ; et
d) séquentiellement solvolyser et soumettre à une réaction de Diels-Alder la 1α-hydroxy 3,5 cyclovitamine D₄ pour former la 1α-hydroxy vitamine D₄.

13. Méthode de production du tosylate de la vitamine D₄ comprenant la réaction de la vitamine D₄ avec du chlorure de toluènesulfonyle en présence de pyridine sèche.

14. Méthode de production de 3,5 cyclovitamine D₄ consistant à soumettre le tosylate de la vitamine D₄ à une solvolyse tamponnée.

15. Méthode de production de 1α-hydroxy 3,5 cyclovitamine D₄, comprenant l'oxydation allylique de la 3,5 cyclovitamine D₄ avec du bioxyde de sélénium.

16. Méthode de production de 1α-hydroxy vitamine D₄ comprenant la solvolyse de la 1α-hydroxy 3,5 cyclovitamine D₄ avec un mélange de diméthylsulfoxyde et d'un acide organique pour former un mélange de 5,6 cis 1α-hydroxy et 5,6 trans 1α-hydroxy vitamine D₄ et consistant à soumettre le mélange à une réaction de Diels-Alder pour former un produit d'addition de la 5,6 trans 1α-hydroxy vitamine D₄ pour donner la 1α-hydroxy vitamine D₄.

17. Méthode de production de 1α-hydroxy vitamine D₄ consistant à :
réduire de l'ergostérol en 22, 23 dihydroergostérol, irradier le 22, 23 dihydroergostérol pour former de la vitamine D₄ et hydroxyler la vitamine D₄ pour former la 1α-hydroxy vitamine D₄.

18. Méthode de production de 1α-hydroxy vitamine D₄ consistant à :
(a) acétyler de l'ergostérol pour former de l'acétate d'ergostéryle ;
(b) hydroxyhalogéner l'acétate d'ergostéryle pour former le 3β-acétoxy-6α-chloroergosta-7,22-dién-5α-ol ;
(c) réduire et réacyler le 3β-acétoxy-6α-ergosta-7,22-dién-5α-ol en 3β-acétoxyergosta-7,22-dien-5α-ol ;
(d) hydrogéner le 3β-acétoxyergosta-7,22-dién-5α-ol pour former du 3β-acétoxyergosto-7-én-5α-ol ;
(e) réduire le 3β-acétoxyergosto-7-én-5α-ol pour former le 22,23 dihydroergostéryl acétate ;
(f) réduire le 22,23 dihydroergostéryl acétate en 22,23 dihydroergostérol ;
(g) irradier le 22,23 dihydroergostérol pour former de la vitamine D₄ ;
(h) tosyler la vitamine D₄ en présence de pyridine sèche pour former de tosylate de vitamine D₄ ;
(i) solvolyser le tosylate de vitamine D₄ pour former la 3,5 cyclovitamine D₄ ;
(j) oxyder allyliquement la 3,5 cyclovitamine D₄ avec du bioxyde de sélénium pour former la 1α-hydroxy vitamine D4 ; et
(k) solvolyser la 1α-hydroxy vitamine D₄ avec un mélange de diméthylsulfoxyde et d'un acide organique pour former un mélange de 5,6 cis 1α-hydroxy et 5,6 trans 1α-hydroxy vitamine D₄ et former un produit d'addition de Diels-Alder de la 5,6 trans 1α-hydroxy vitamine D₄ pour donner la 1α-hydroxy vitamine D₄.

19. Alimentation pour mammifère comprenant au moins un composé de la formule (I') tel qu'indiqué à la revendication 9 où R₁ est soit H ou OH et R₂ est soit H ou OH, où la consommation normale de l'alimentation par les mammifères procure environ 0,01 à environ 0,5 µg/kg/jour dudit composé.

20. Utilisation d'un composé de formule (I') tel qu'indiqué à la revendication 9 pour la fabrication d'un médicament pour le traitement d'une maladie induite par une déficience en vitamine D,
et/ou de l'ostéoporose,
et/ou de troubles hyperprolifératifs de la peau,
et/ou d'hypocalcémie,
et/ou pour contrôler le métabolisme du calcium chez un mammifère.

21. Utilisation selon la revendication 20, où le médicament est pour administration orale, intramusculaire ou intraveineuse.

22. Utilisation selon la revendication 20 ou la revendication 21, où le médicament est administré par dosage quotidien de 0,04 µg à 1,5 µg par kg de poids corporel du mammifère traité.

23. Utilisation selon la revendication 20 ou la revendication 21 ou la revendication 22, où l'hypocalcémie est le rachitisme dépendant de la vitamine D, l'hypoparathyroïdie post-opératoire, l'ostéodystrophie rénale, la cirrhose du foie ou la stéatorrhée.

24. Méthode de production de 1,24 dihydroxy vitamine D₄ comprenant l'incubation de 1α-hydroxy vitamine D₄ avec des cellules de foie humain, la culture des cellules et la récupération de la 1,24 dihydroxy vitamine D₄.

25. Méthode de préparation d'une composition pharmaceutique prophylactique ou thérapeutique, comprenant le mélange d'une quantité d'un composé de la formule (I') avec une substance de support pharmaceutiquement acceptable appropriée pour application entérale, parentérale ou topique : 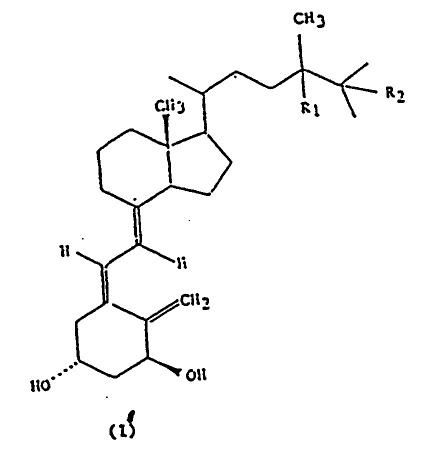 où R₁ est H ou OH et R₂ est H ou OH, en combinaison avec un véhicule pharmaceutiquement acceptable.
